# EUROPEAN PATENT SPECIFICATION

(11) **EP 0 953 624 B1**
(45) Date of publication and mention of the grant of the patent: **04.02.2004**
(21) Application number: 99303199.6
(22) Date of filing: 26.04.1999
(51) Int. Cl.: C09K 11/06, H05B 33/14, C07C 211/54

(54) **Triarylamine compound and luminescent device**
Triarylaminverbindungen und lumineszente Vorrichtung
Composés du type triarylamine et dispositif luminescent

(30) Priority: 28.04.1998 JP 13263698
(43) Date of publication of application: 03.11.1999
(73) Proprietor: CANON KABUSHIKI KAISHA, Tokyo (JP)
(72) Inventor: Senoo, Akihiro, Ohta-ku, Tokyo (JP); Ueno, Kazunori, Ohta-ku, Tokyo (JP); Urakawa, Shinichi, Ohta-ku, Tokyo (JP); Hashimoto, Yuichi, Ohta-ku, Tokyo (JP); Mashimo, Seiji, Ohta-ku, Tokyo (JP)
(74) Representative: Beresford, Keith Denis Lewis

(56) References cited:
- EP-A- 0 879 868
- US-A- 4 539 507
- US-A- 4 720 432
- CHEMICAL ABSTRACTS, vol. 128, no. 26, 29 June 1998 (1998-06-29) Columbus, Ohio, US; abstract no. 328604, NAKATSUKA, MASAKATSU ET AL: "Organic electroluminescent device" XP002110838 & JP 10 095972 A (MITSUI TOATSU CHEMICALS, INC., JAPAN) 14 April 1998 (1998-04-14)
- PATENT ABSTRACTS OF JAPAN vol. 018, no. 591 (C-1272), 11 November 1994 (1994-11-11) & JP 06 220437 A (RICOH CO LTD;OTHERS: 01), 9 August 1994 (1994-08-09)
- PATENT ABSTRACTS OF JAPAN vol. 098, no. 002, 30 January 1998 (1998-01-30) & JP 09 268284 A (TOYO INK MFG CO LTD), 14 October 1997 (1997-10-14)
- PATENT ABSTRACTS OF JAPAN vol. 095, no. 005, 30 June 1995 (1995-06-30) & JP 07 053950 A (TOYO INK MFG CO LTD), 28 February 1995 (1995-02-28)
- PATENT ABSTRACTS OF JAPAN vol. 017, no. 309 (C-1070), 14 June 1993 (1993-06-14) & JP 05 025473 A (MITSUI TOATSU CHEM INC), 2 February 1993 (1993-02-02)

## Description

### Field of the Invention

The present invention relates to a triarylamine compound and to a charge-injection-type luminescent device using the same. In particular, the present invention relates to a triarylamine compound applicable to a charge-injection-type luminescent device which directly converts injected charges into optical energy by an applied electric field, and relates to a luminescent device using the same.

### Description of the Related Art

Pope et al., first discovered electroluminescence (EL) in an organic material, that is, single-crystal anthracene in 1963 (*J. Chem. Phys*., 38, 2042 (1963)). Subsequently, Helfinch and Schneider observed relatively strong EL in an injection EL material containing a solution system having a high injection efficiency in 1965 (*Phys. Rev. Lett*., 14, 229 (1965)).

Many studies of organic luminescent materials containing conjugated organic hosts and conjugated organic activators having condensed benzene rings have been disclosed in U.S. Patent Nos. 3,172,862, 3,173,050, and 3,710,167; *J. Chem. Phys*., 44, 2902 (1966); *J. Chem. Phys*., 58, 1542 (1973); and *Chem. Phys. Lett*., 36, 345 (1975). Examples of disclosed organic hosts include naphthalene, anthracene, phenanthrene, tetracene, pyrene, benzpyrene, chrysene, picene, carbazole, fluorene, biphenyl, terphenyl, triphenylene oxide, dihalobiphenyl, trans-stilbene, and 1,4-diphenylbutadiene. Examples of disclosed activators include anthracene, tetracene and pentacene. Since these organic luminescent materials are provided as single layers having a thickness of more than 1 µm, a high electric field is required for luminescence. Under these circumferences, thin film devices formed by a vacuum deposition process have been proposed (for example, "Thin Solid Films" p. 94 (1982); *Polymer*, 24, 748 (1983); and *J. Appl. Phys.,* 25, L773 (1986)). Although the thin film devices are effective for reducing the driving voltage, their luminance is far from levels for practical use.

In recent years, Tang, et al., have developed an EL device having a high luminance at a low driving voltage (*Appl. Phys. Lett*., 51, 913 (1987) and U.S. Patent No. 4,356,429). The EL device is fabricated by depositing two significantly thin layers, that is, a charge transport layer and a luminescent layer, between the anode and the cathode by a vacuum deposition process. Such layered organic EL devices are disclosed in, for example, Japanese Patent Application Laid-Open Nos. 59-194393, 59-194393, 63-264692, and 3-163188, U.S. Patent Nos. 4,539,507 and 4,720,432, and *Appl. Phys. Lett*., 55, 1467 (1989).

Also, an EL device of a triple-layered structure having independently a carrier transport function and a luminescent ability was disclosed in *Jpn. J. Apply. Phys*., 27, L269 and L713 (1988). Since the carrier transportability is improved in such an EL device, the versatility of possible dyes in the luminescent layer is considerably increased. Further, the device configuration suggests feasibility of improved luminescence by effectively trapping holes and electrons (or excimers) in the central luminescent layer.

Layered organic EL devices are generally formed by vacuum deposition processes. EL devices having considerable luminance are also formed by casting processes (as described in, for example, Extended Abstracts (The 50th Autumn Meeting (1989), p. 1006 and The 51st Autumn Meeting (1990), p. 1041; The Japan Society of Applied Physics). Considerably high luminance is also achieved by a single-layered mixture-type EL device, in which the layer is formed by immersion-coating a solution containing polyvinyl carbazole as a hole transport compound, an oxadiazole derivative as an electron transport compound and coumarin-6 as a luminescent material, as described in Extended Abstracts of the 38th Spring Meeting 1991, p. 1086; The Japan Society of Applied Physics and Related Societies.

As described above, the organic EL devices have been significantly improved and have suggested the feasibility of a wide variety of applications; however, these EL devices have some problems in practical use, for example, insufficient luminance, changes in luminance during prolonged use, and deterioration by atmospheric gas containing oxygen and humidity. Further, the EL devices do not sufficiently satisfy needs for diverse wavelengths of luminescent light for precisely determining luminescent hues of blue, green and red colors in full-color displays, etc.

Patent Abstracts of Japan, vol.018, No. 591 (C-1272), 11 November 1994 and JP 06 220437 disclose an electroluminescent element having long-lasting luminescent properties and excellent durability achieved by incorporating a specific compound into at least one organic compound layer. Among the specific compounds disclosed are triaryl diamine compounds in wh ich the two amine groups are linked by an optionally substituted fluorene group. Each of the amine nitrogen atoms has a pyrene substituent.

### SUMMARY OF THE INVENTION

It is an object of the present invention to provide an organic compound applicable to a luminescent device having an optical output with significantly high efficiency and luminance.

It is another object of the present invention to provide an organic compound applicable to a luminescent device, which has diverse luminescent wavelengths, a variety of luminescent hues, and significantly high durability.

It is a further object of the present invention to provide a luminescent device easily produced at relatively low production cost and high safety.

An aspect of the present invention is a triarylamine compound represented by the following general formula [1]: 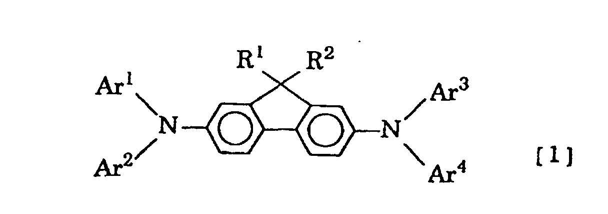 wherein:
R¹ and R² are each independently a hydrogen atom, a halogen atom, a substituted or unsubstituted alkyl group, a substituted or unsubstituted alkoxy group, or a substituted or unsubstituted aryl group,
Ar¹, Ar², Ar³ and Ar⁴ are each a substituted or unsubstituted aryl or heterocyclic group which may be the same or different from each other selected from phenyl, biphenyl, terphenyl, naphthyl, anthryl, penanthryl, pyridyl, furyl, thienyl and carbazolyl;
and at least one of Ar¹, Ar², Ar³, and Ar⁴ is a fused aromatic ring selected from naphthyl, anthryl, acenaphthyl, phenanthryl, naphthathenyl and fluoranthenyl.

Another aspect of the present invention is a triarylamine compound represented by the following general formula [2]: 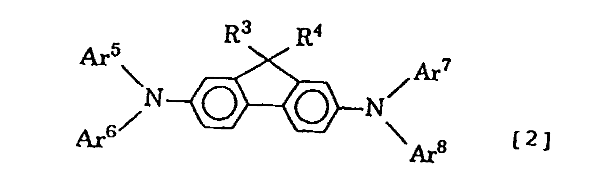 wherein:
R³ and R⁴ are each independently a hydrogen atom, a halogen atom, a substituted or unsubstituted alkyl group, a substituted or unsubstituted alkoxy group, or a substituted or unsubstituted aryl group;
Ar⁵, Ar⁶, Ar⁷ and Ar⁸ are each a substituted or unsubstituted aryl or heterocyclic group, which may be the same or different from each other selected from phenyl, biphenyl, terphenyl, naphthyl, anthryl, penanthryl, pyridyl, furyl, thienyl and carbazolyl; and at least one of Ar⁵, Ar⁶, Ar⁷ and Ar⁸ is a π-conjugated aromatic hydrocarbon having 12 or more carbon atoms selected from polyphenyls and stilbene derivatives.

A further aspect of the present invention is a luminescent device comprising a pair of electrodes, and at least one compound among the compounds represented by the general formulae [1] and [2] disposed therebetween.

The organic luminescent device in accordance with the present invention is a thin lightweight solid device having a large area and high resolution and capable of high-speed operation, unlike conventional incandescent lamps, fluorescent lamps, and inorganic luminescent diodes, and thus satisfies advanced requirements.

### BRIEF DESCRIPTION OF THE DRAWINGS

Fig. 1 is a cross-sectional view of an embodiment of a luminescent device in accordance with the present invention;
Fig. 2 is a cross-sectional view of another embodiment of a luminescent device in accordance with the present invention;
Fig. 3 is a cross-sectional view of a further embodiment of a luminescent device in accordance with the present invention;
Fig. 4 is a cross-sectional view of a luminescent device in accordance with Example 7 of the present invention; and
Fig. 5 is an infrared spectrum of an organic compound in accordance with the present invention.

### DESCRIPTION OF THE PREFERRED EMBODIMENT

The present invention is characterized by a novel triarylamine compound represented by the general formula [1] or [2]:

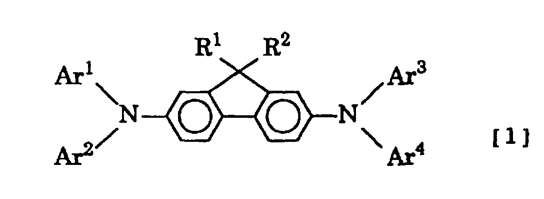

In the general formula [1], R¹ and R² are each independently a hydrogen atom, a halogen atom, a substituted or unsubstituted alkyl group, a substituted or unsubstituted alkoxy group, or a substituted or unsubstituted aryl group.

Examples of alkyl groups include methyl, ethyl, n-propyl, and isopropyl groups; examples of alkoxy groups include methoxy, ethoxy, and phenoxy groups; and examples of aryl groups include phenyl, biphenyl, and naphthyl groups.

Examples of the substituent groups include halogen atoms, e.g., fluorine, chlorine, bromine, and iodine; alkyl groups, e.g,. methyl, ethyl, n-propyl and iso-propyl groups; alkoxy groups, e.g., methoxy, ethoxy, and phenoxy groups; aralkyl groups, e.g., benzyl, phenethyl, and propylphenyl group; a nitro group; a cyano group; substituted amino groups, e.g., dimethyl amino, dibenzylamino, diphenylamino, and morpholino groups; aryl groups, e.g., phenyl, tolyl, biphenyl, naphthyl, anthryl, and pyrenyl groups; and heterocyclic groups, e.g., pyridyl, thienyl, furyl, quinolyl, and carbazolyl groups.

In the general formula [1], Ar¹, Ar², Ar³, and Ar⁴ are each a substituted or unsubstituted aryl or heterocyclic group, which may be the same or different from each other selected from phenyl, biphenyl, terphenyl, naphthyl, anthryl, phenanthryl, pyridyl, furyl, thienyl, and carbazolyl groups.

At least one of Ar¹, Ar², Ar³, and Ar⁴ is a fused aromatic ring selected from naphthyl, anthryl, acenaphthyl, phenanthryl, naphthathenyl and fluoranthenyl rings. These fused aromatic rings may have substituent groups. Examples of the substituent groups include halogen atoms, e.g., fluorine, chlorine, bromine, and iodine; alkyl groups, e.g. methyl, ethyl, n-propyl, and iso-propyl groups; alkoxy groups, e.g., methoxy, ethoxy, and phenoxy groups; aralkyl groups, e.g., benzyl, phenethyl, and propylphenyl group; a nitro group; a cyano group; substituted amino groups, e.g., dimethyl amino, dibenzylamino, diphenylamino, and morpholino groups; aryl groups, e.g., phenyl, tolyl, biphenyl, naphthyl, anthryl, and pyrenyl groups; and heterocyclic groups, e.g., pyridyl, thienyl, furyl, quinolyl, and carbazolyl groups.

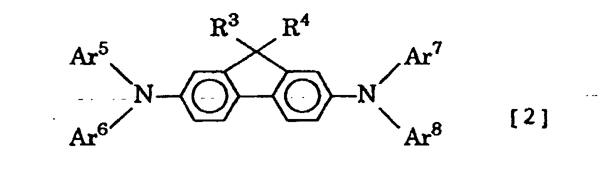

In the general formula [2], R³ and R⁴ are the same as R¹ and R², respectively, in the general formula [1], and Ar⁵, Ar⁶, Ar⁷, and Ar⁸ are the same as Ar¹, Ar², Ar³, and Ar⁴, respectively, in the general formula [1]. At least one of Ar⁵, Ar⁶, Ar⁷, and Ar⁸ is a π-conjugated aromatic hydrocarbon having 12 or more carbon atoms selected from polyphenyls, i.e., biphenyl, p-terphenyl, and quaterphenyl; and stilbene derivatives, i.e., styryl and phenylstyryl.

The following are typical non-limiting examples of the compounds represented by the general formula [1] or [2].

Compounds represented by the general formula [1]

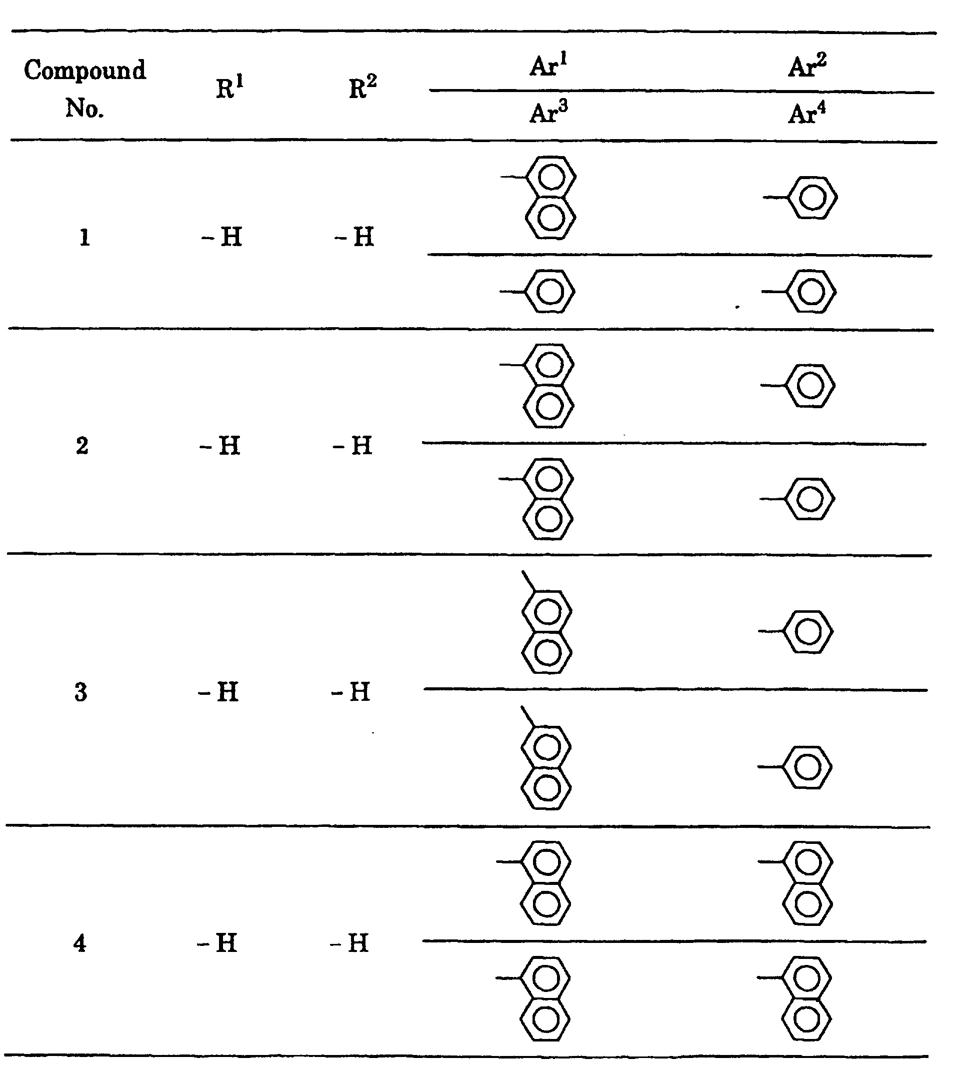

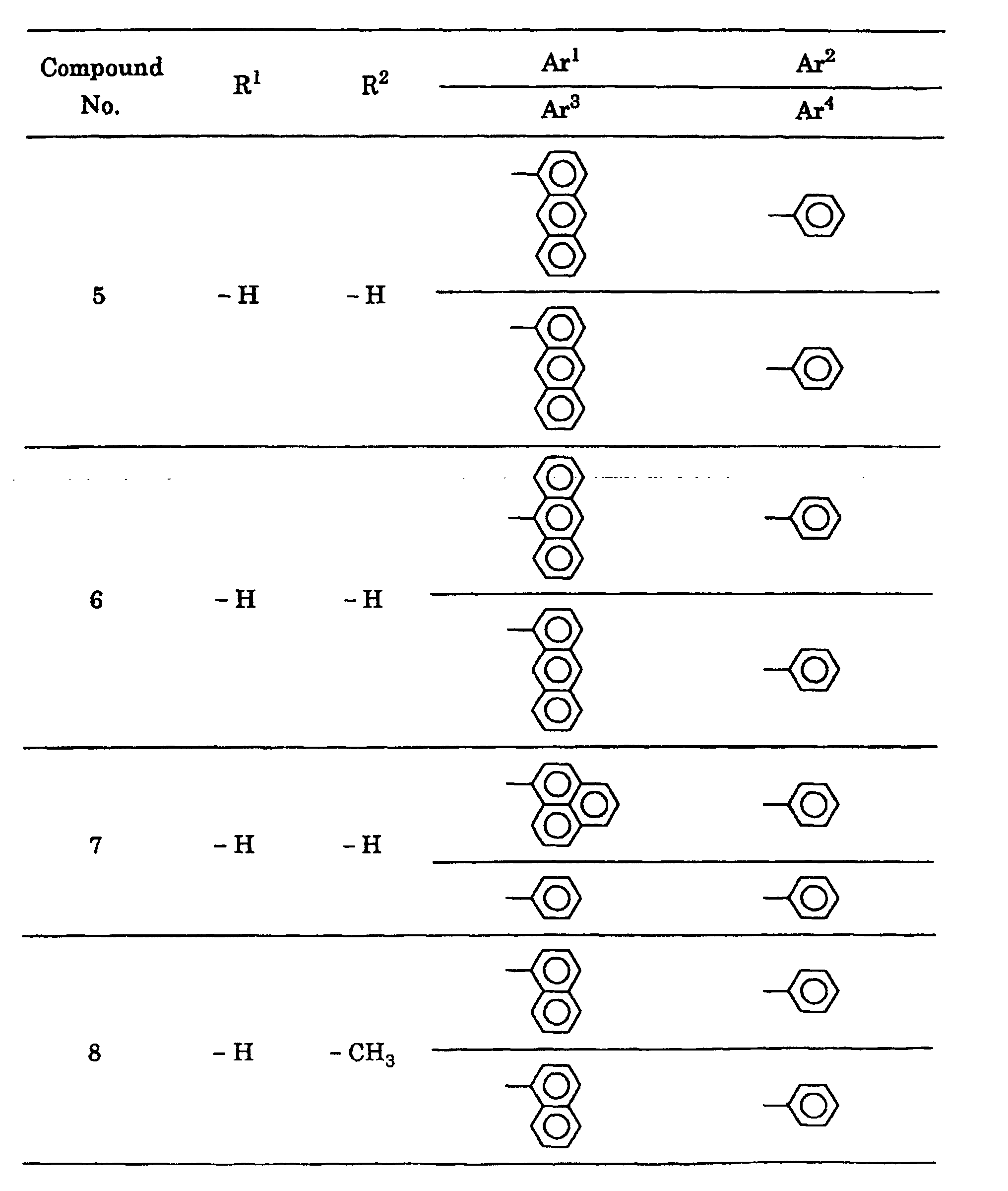

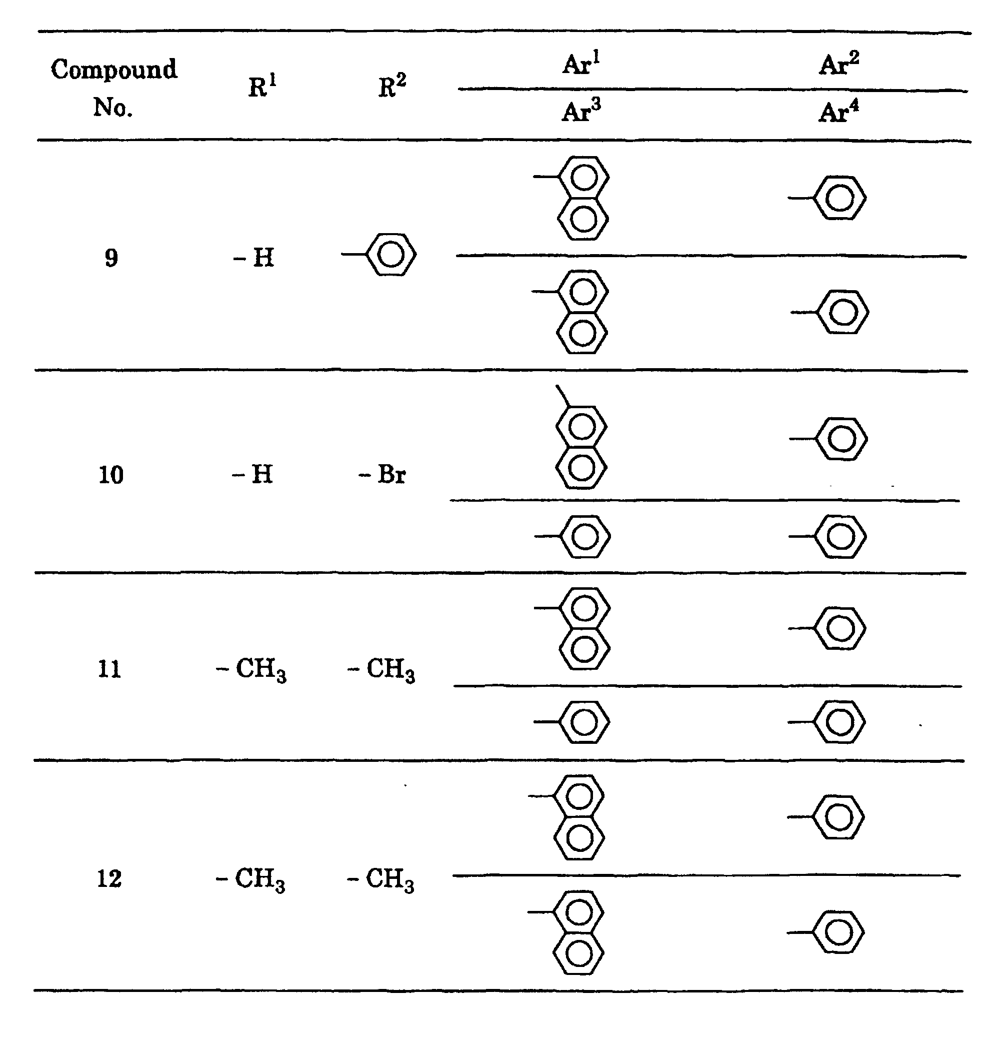

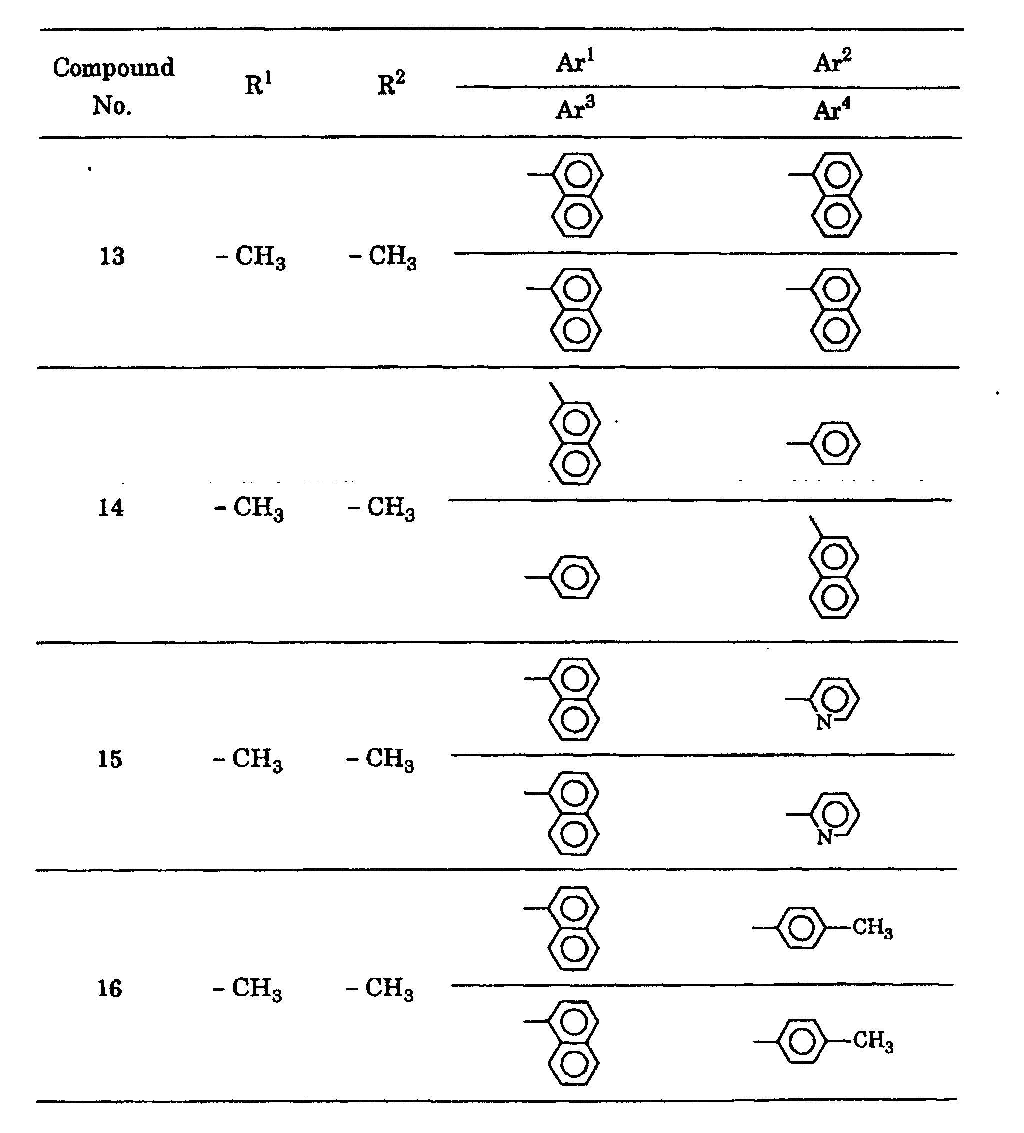

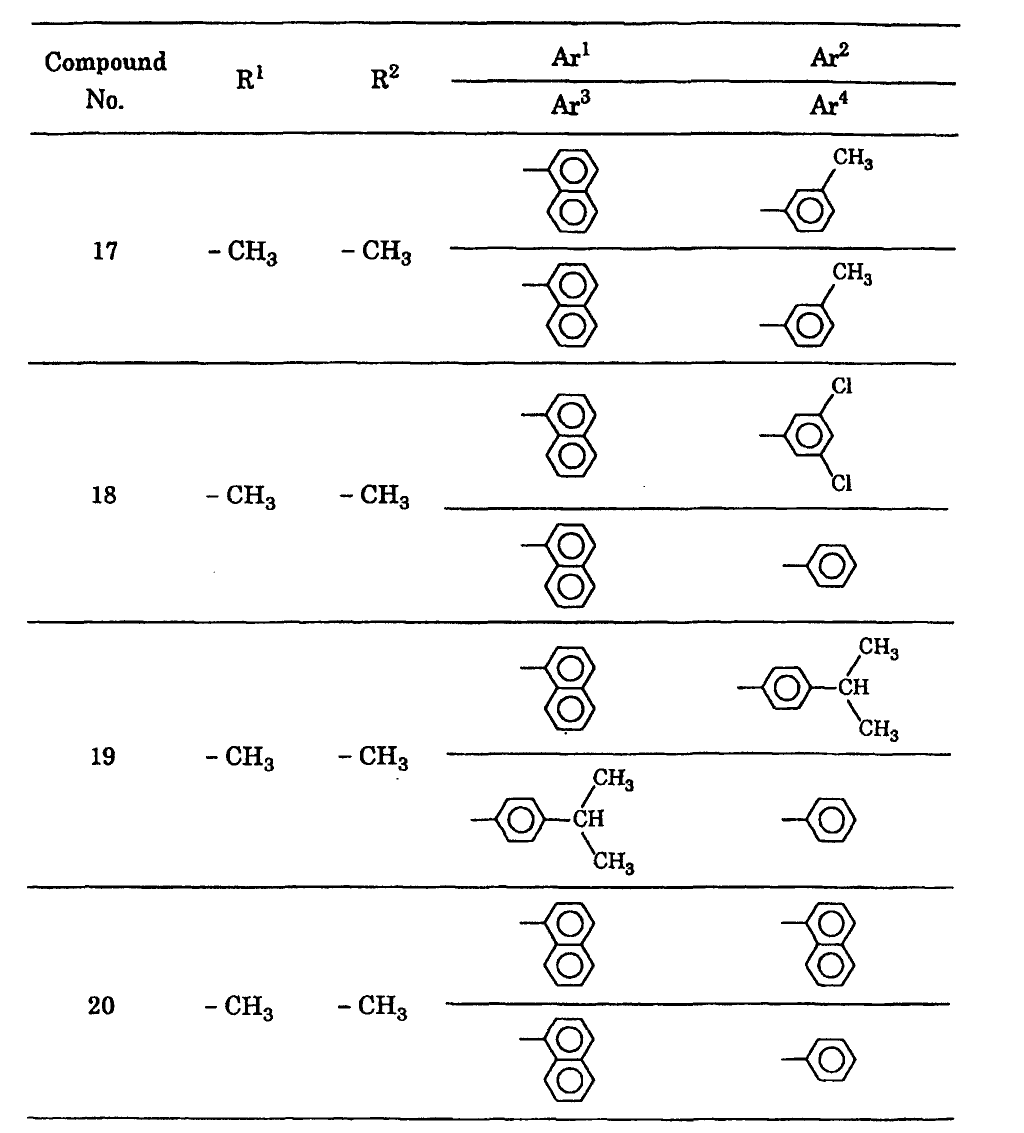

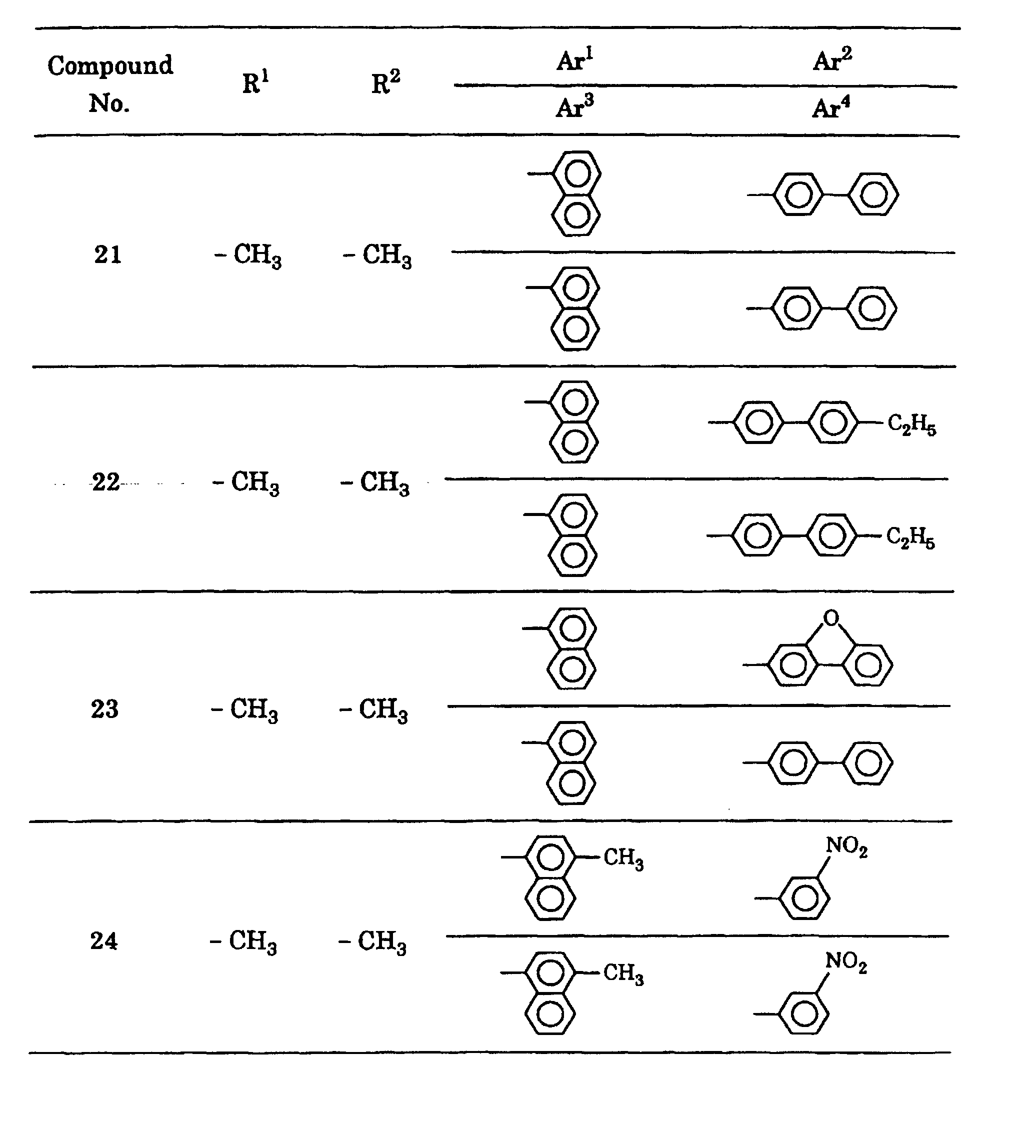

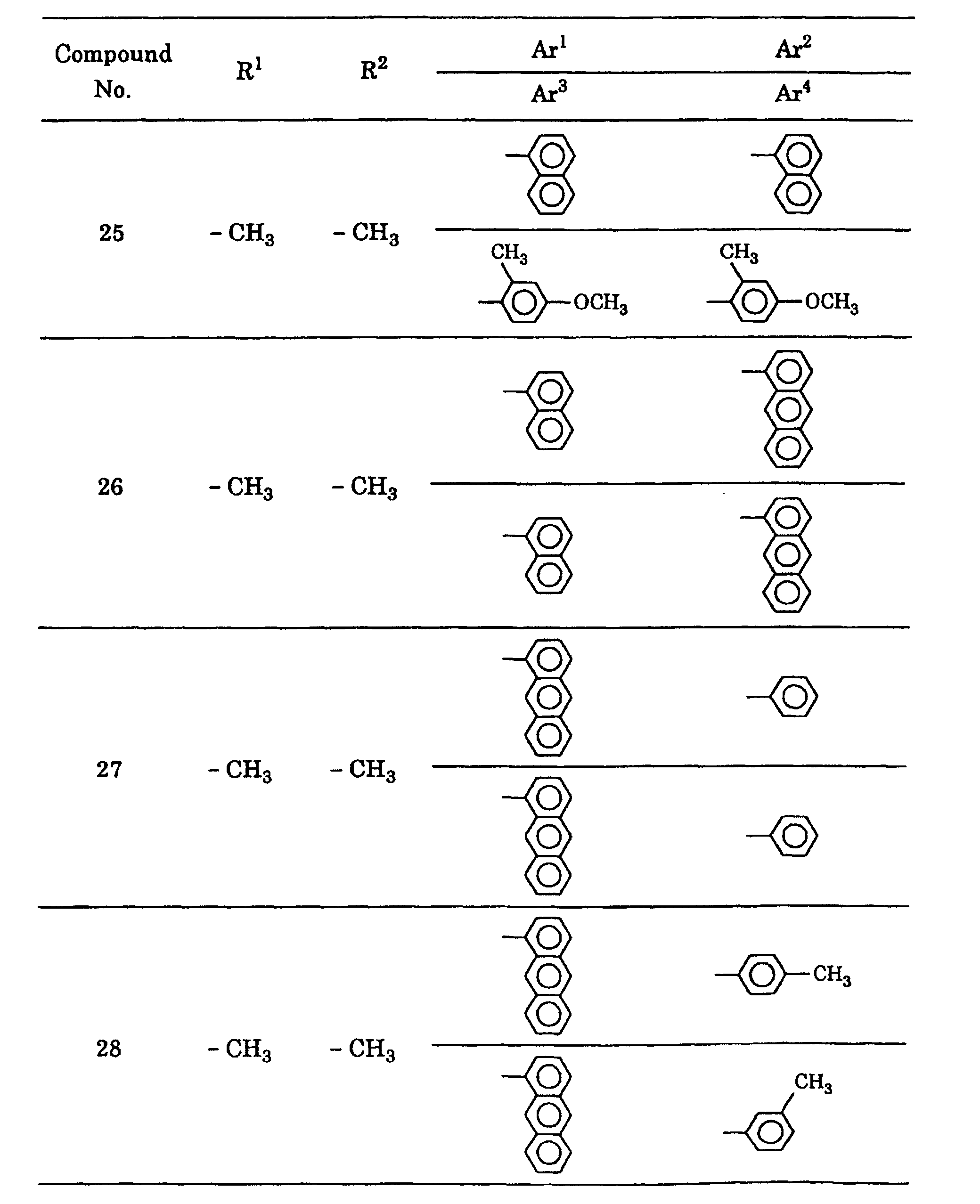

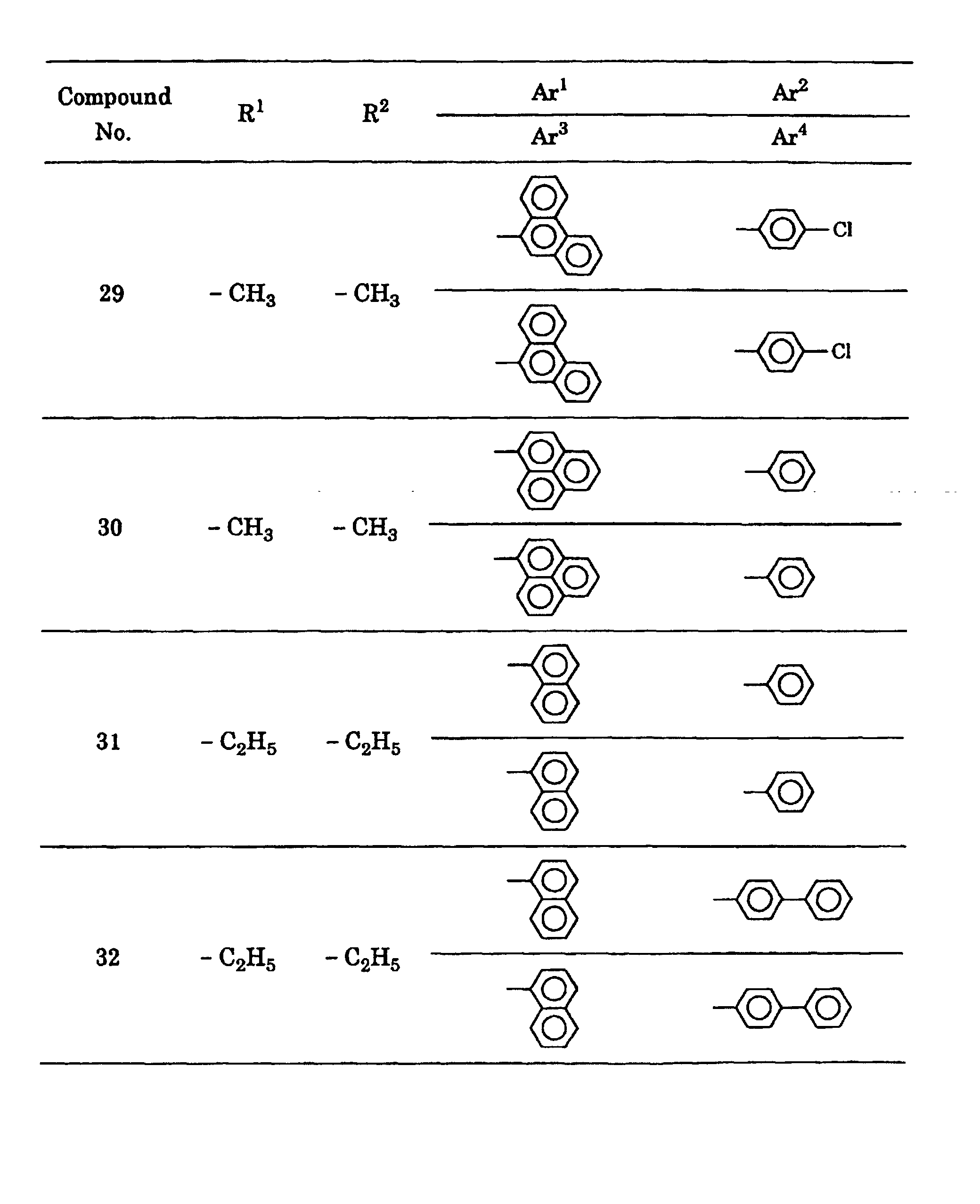

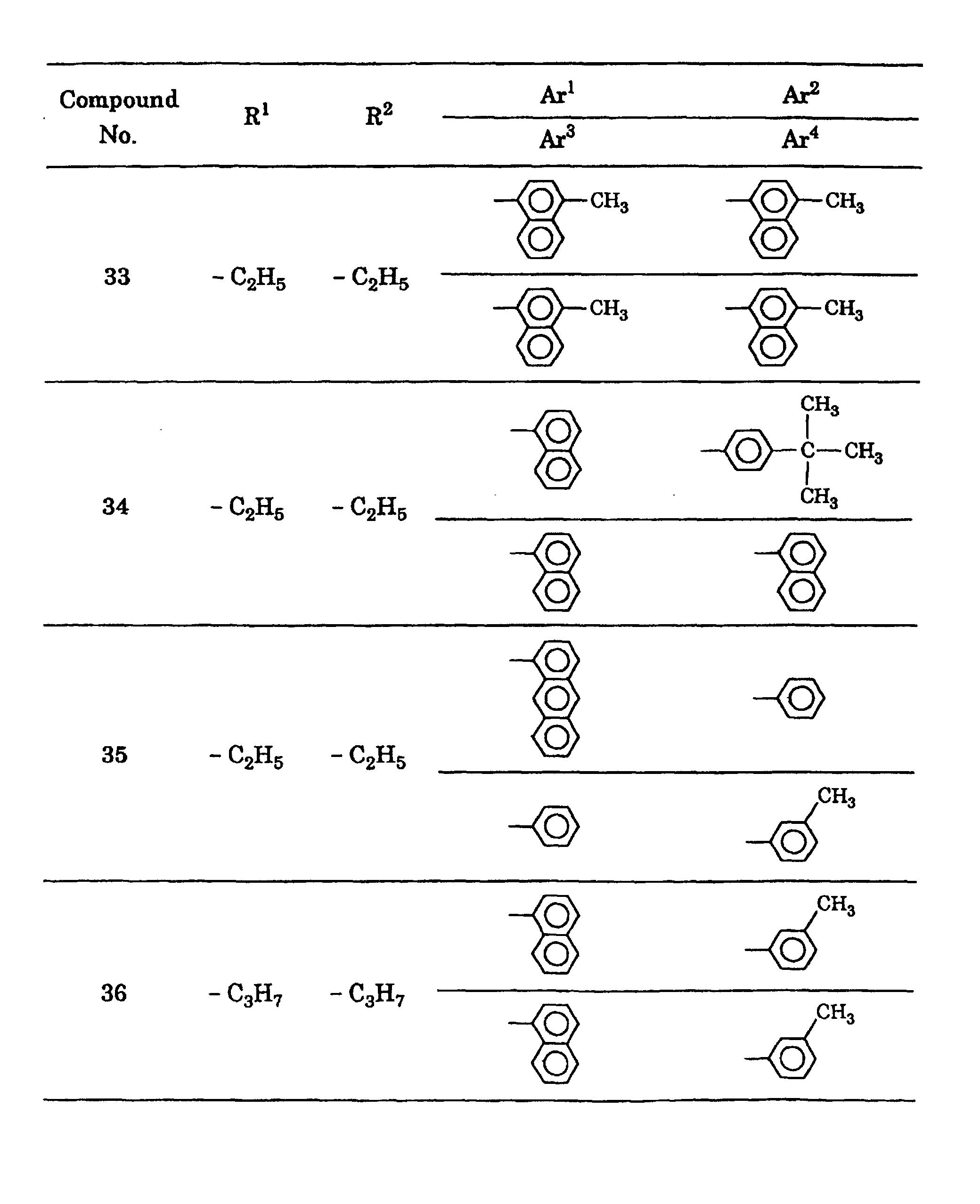

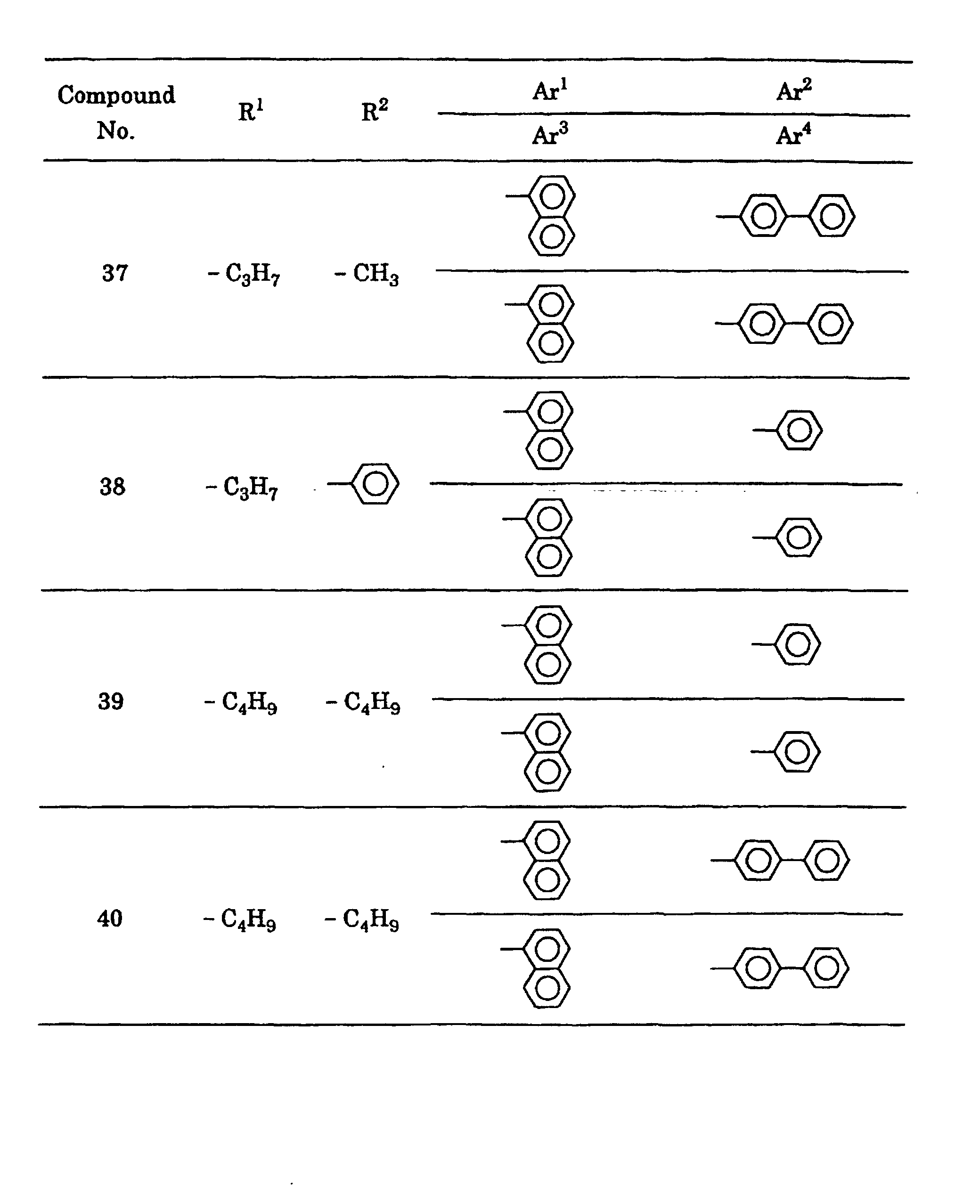

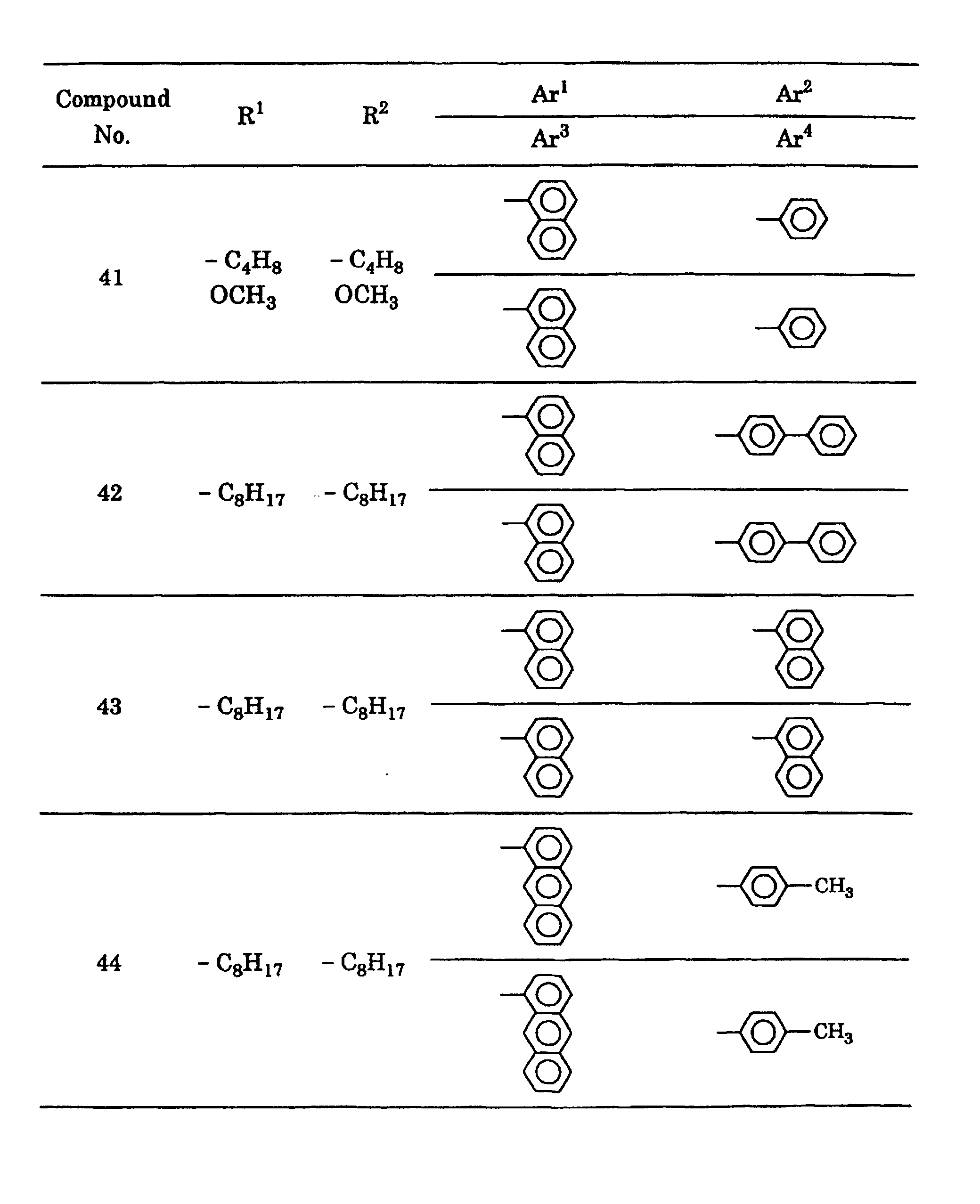

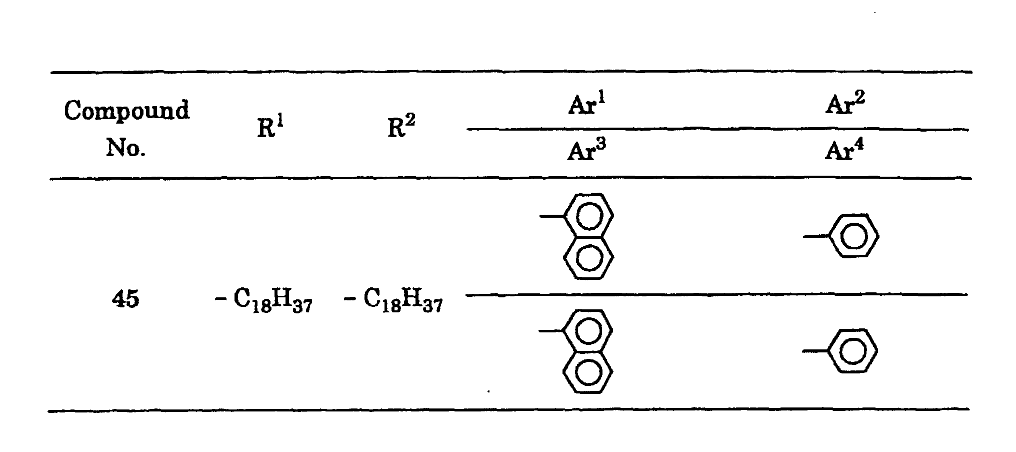

Compounds represented by the general formula [2]

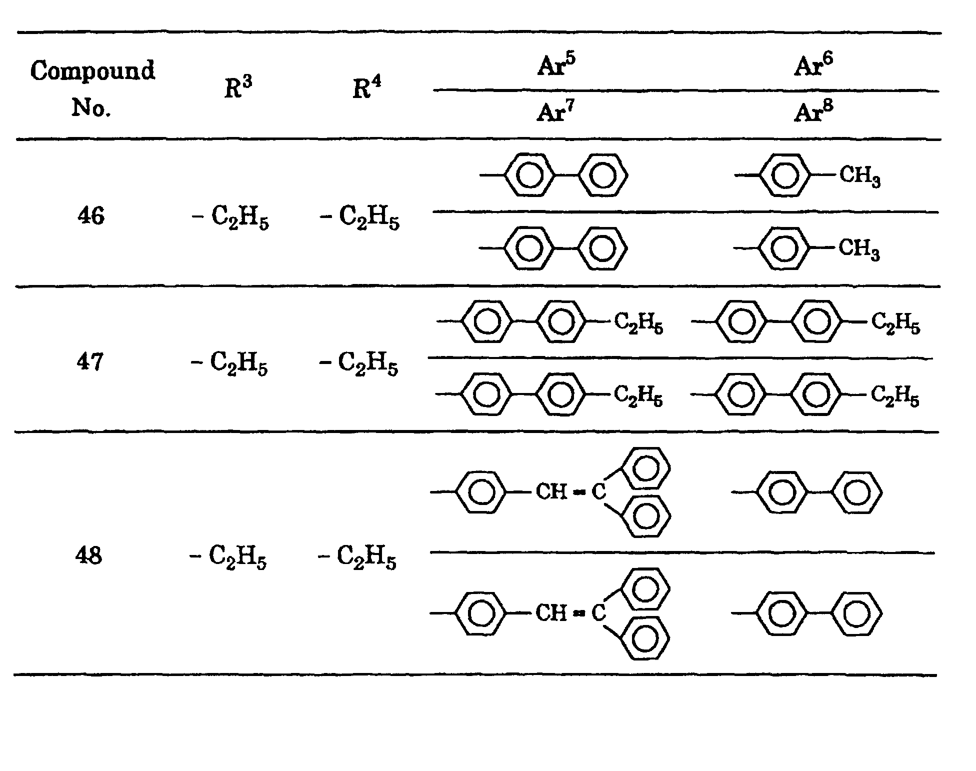

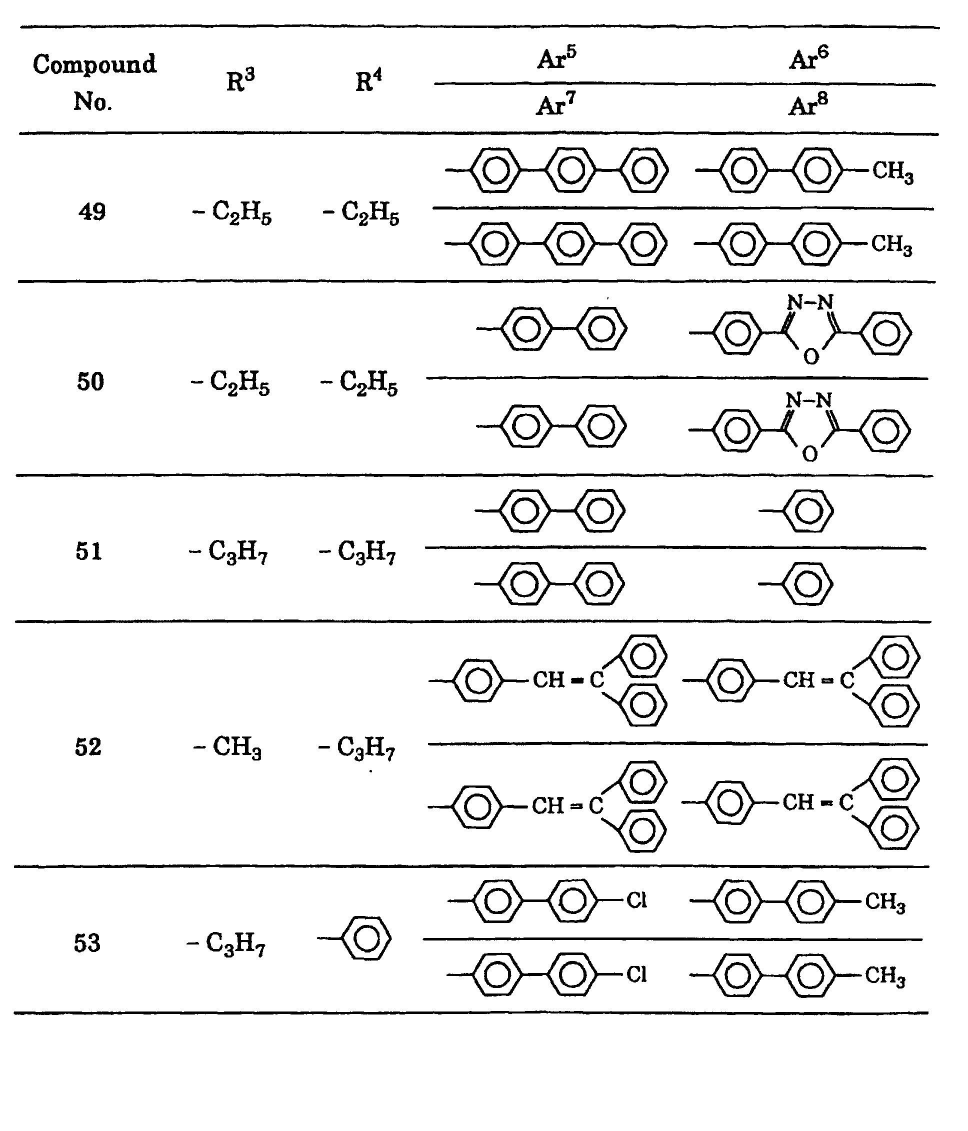

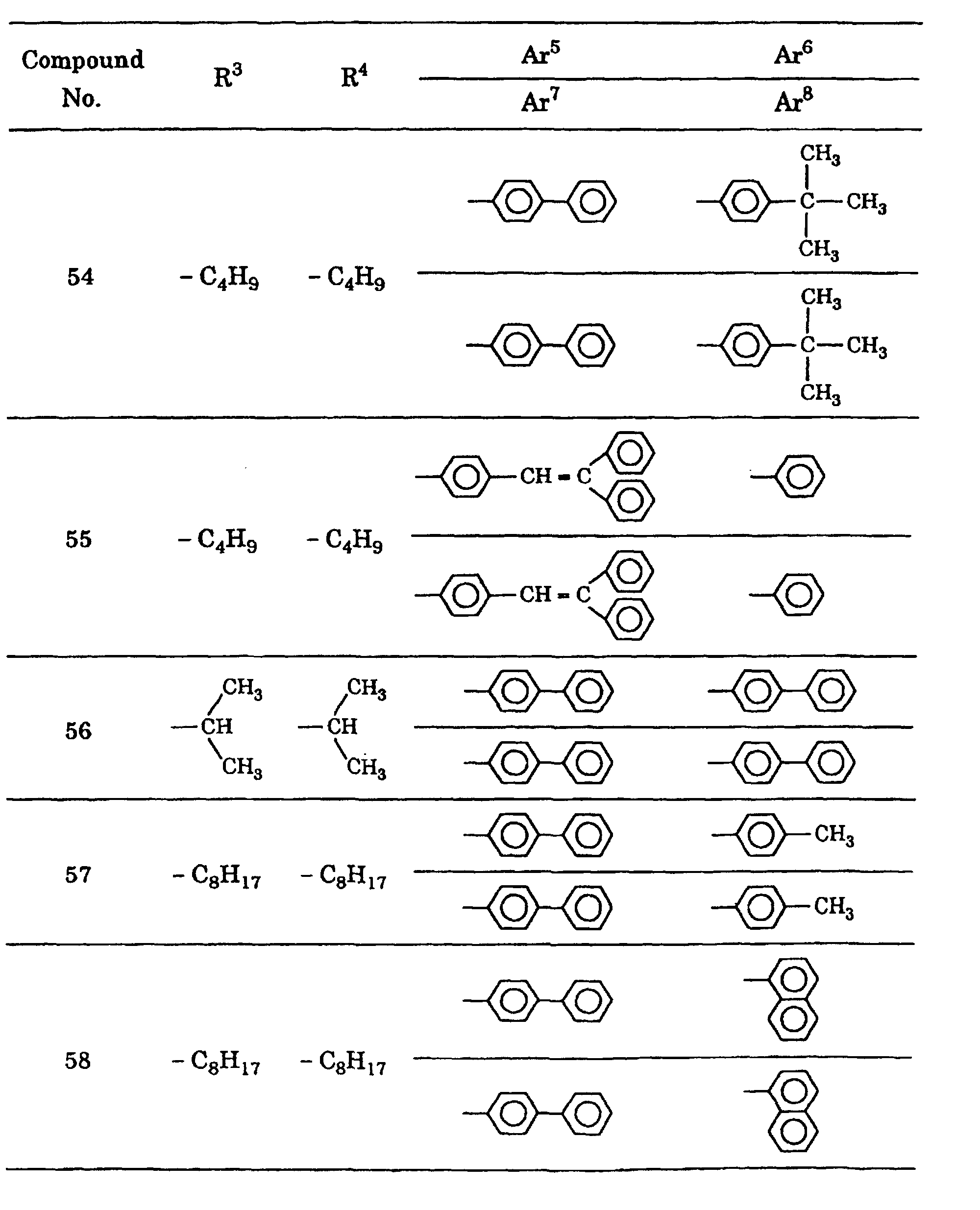

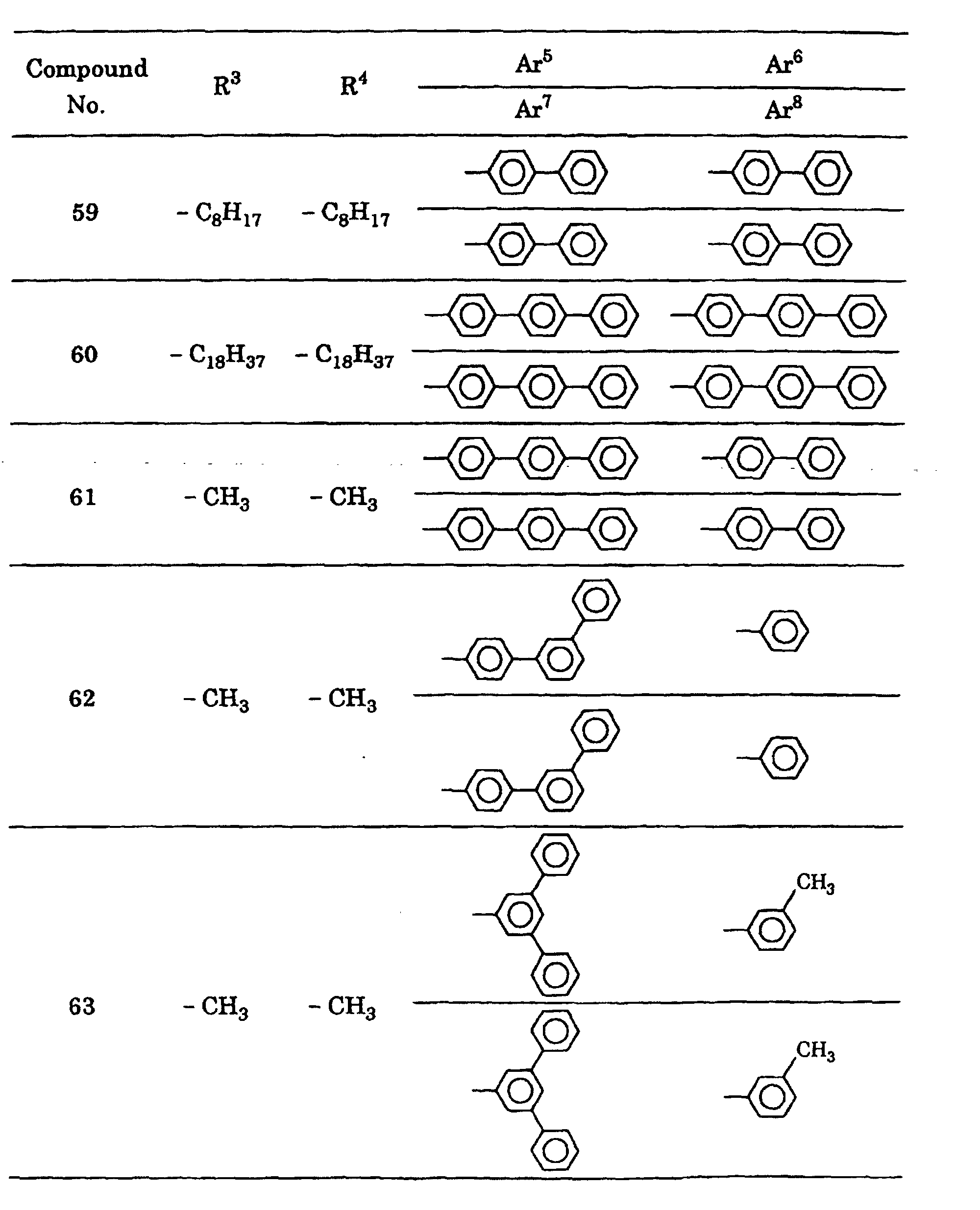

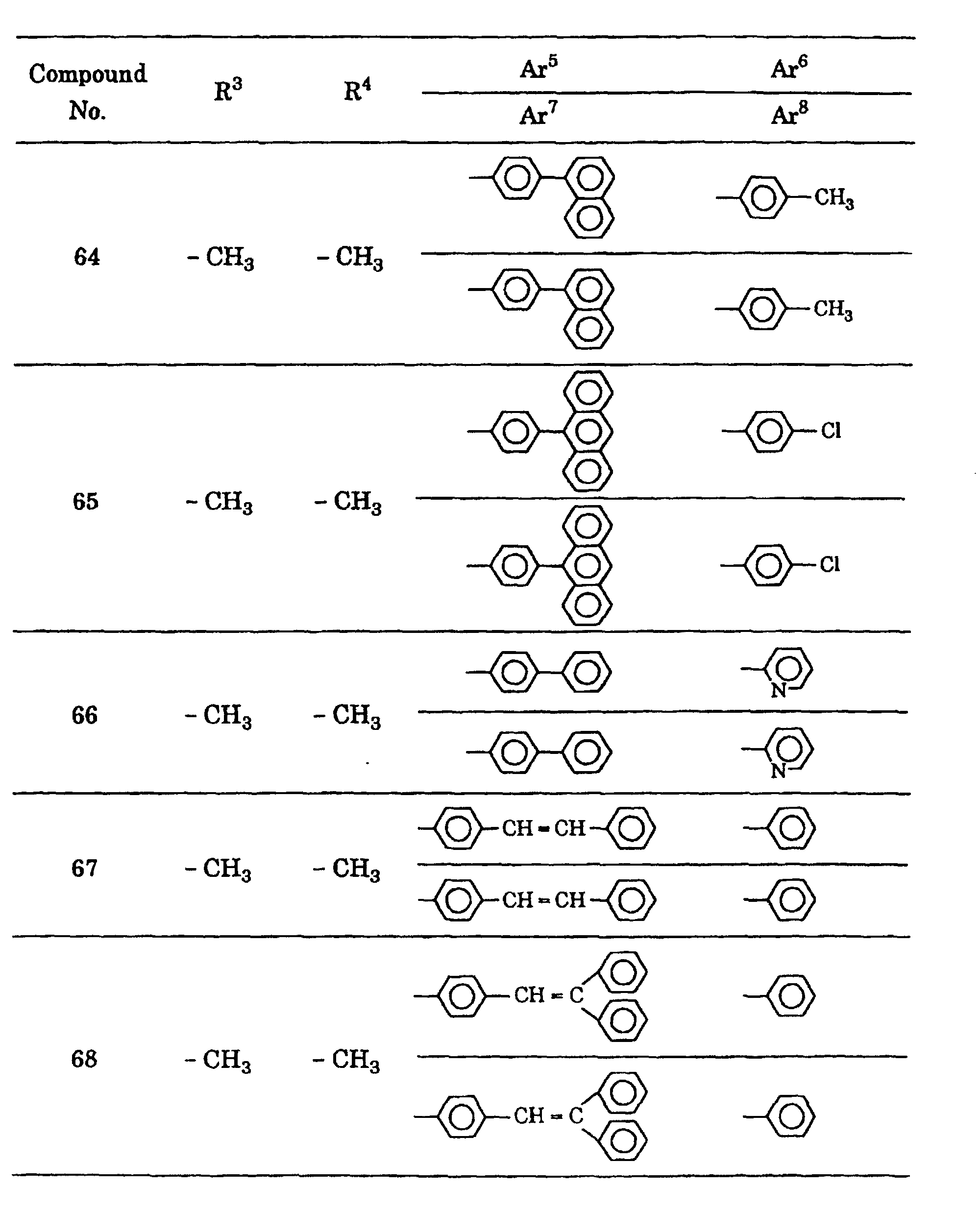

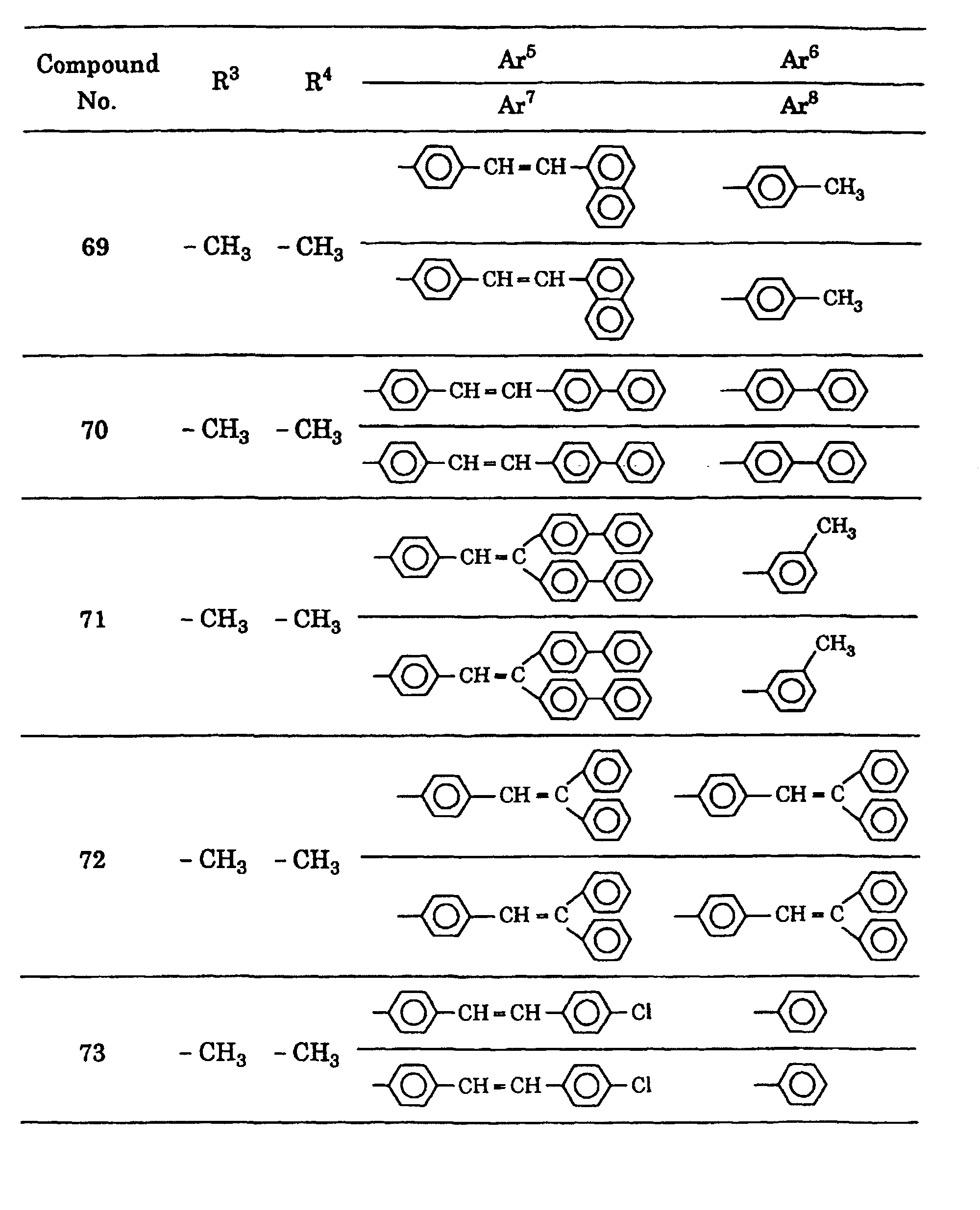

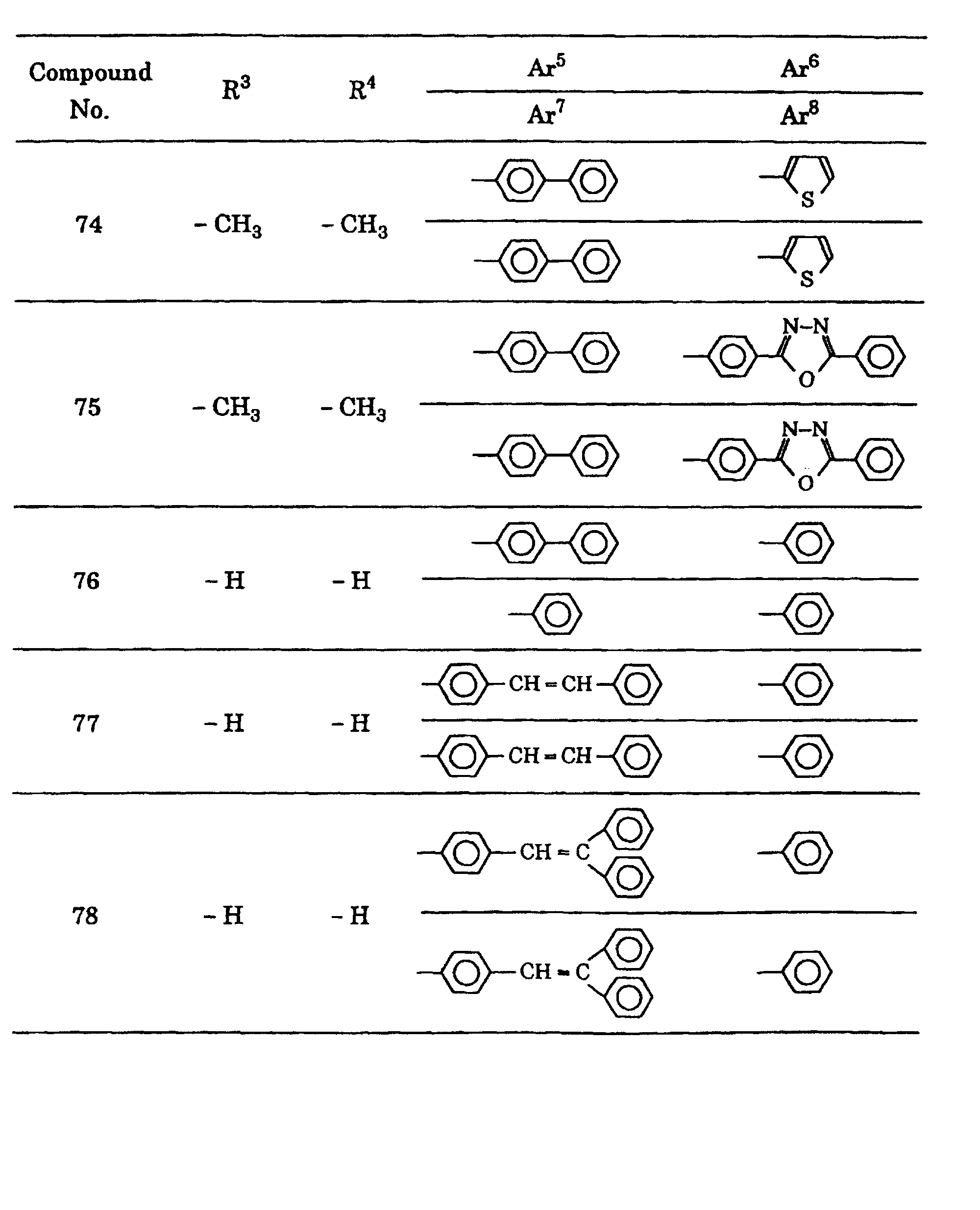

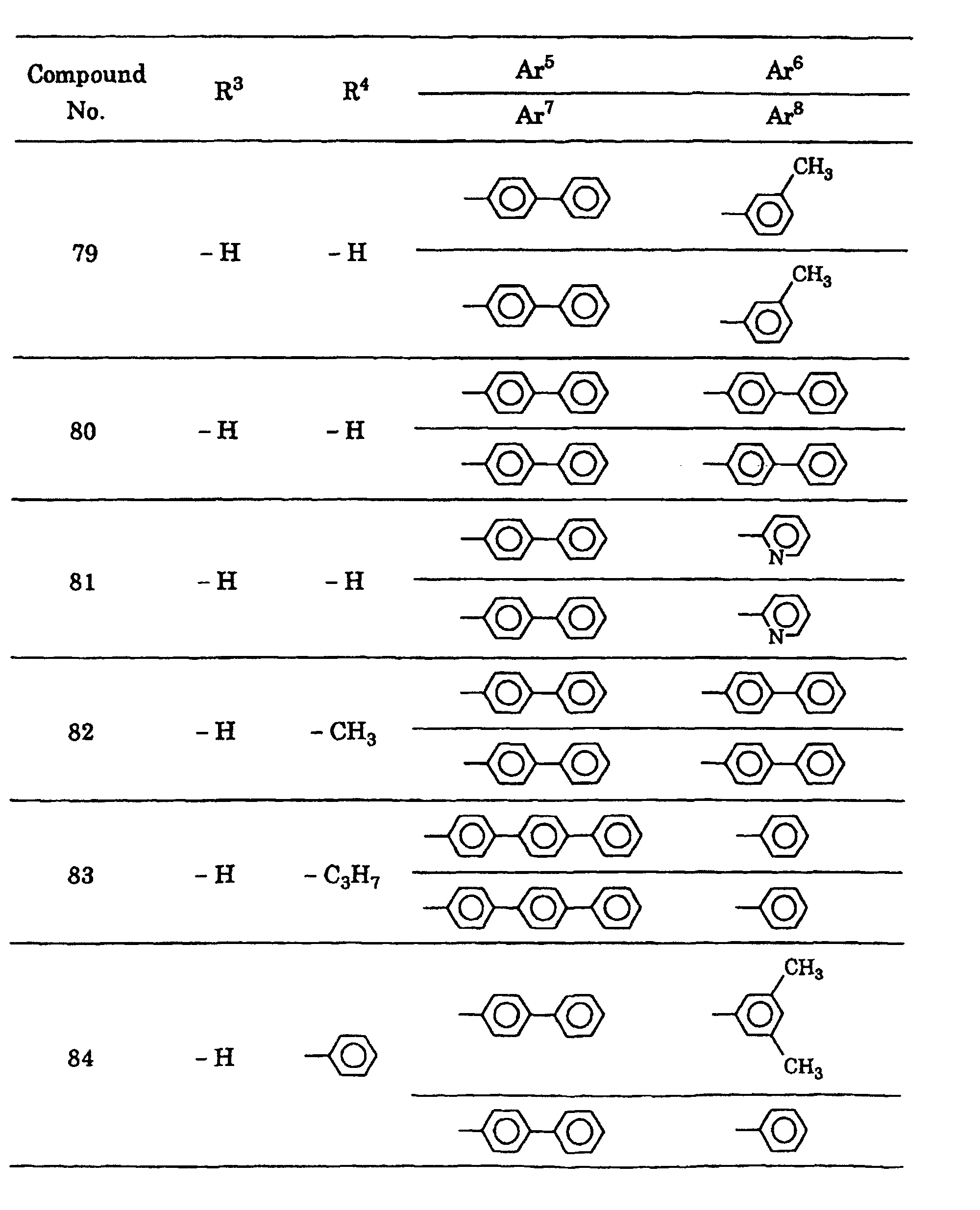

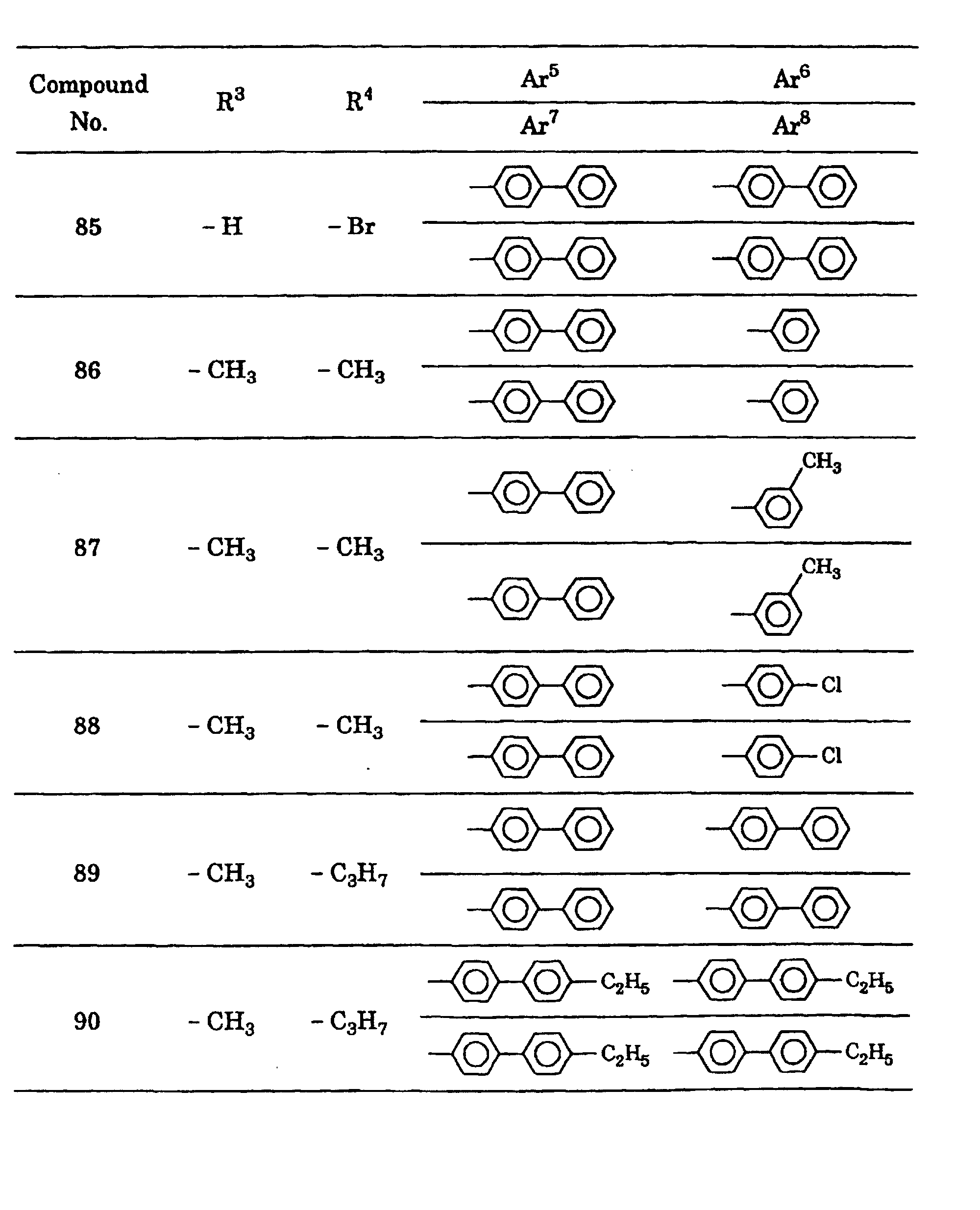

The luminescent device in accordance with the present invention has a layer or a plurality of layers composed of an organic compound disposed between an anode and a cathode, and at least one layer among the above organic layers contains a compound represented by the general formula [1] or [2].

The layer of the organic compound represented by the general formula [1] or [2] is formed between the anode and the cathode by a vacuum deposition process or a solution coating process. The thickness of the organic layer is preferably 2 µm or less, and more preferably 0.5 µm or less, and most preferably 0.05 to 0.5 µm.

The present invention will now be described in further detail with reference to the drawings.

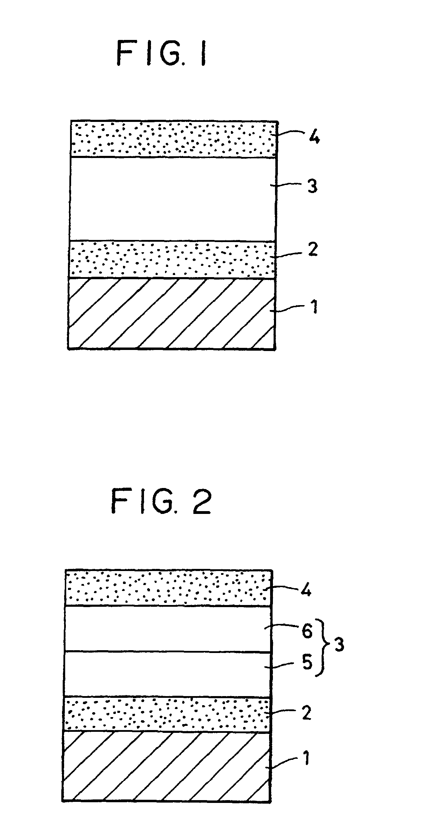

Fig. 1 is a schematic cross-sectional view of an embodiment of the luminescent device in accordance with the present invention. An anode 2, a luminescent layer 3 and a cathode 4 are formed on a substrate 1, in that order. In such a configuration, a usable luminescent layer 3 is generally composed of a single compound having hole transportability, electron transportability and luminescence, or a mixture of compounds each having one of these properties.

Fig. 2 is a schematic cross-sectional view of another embodiment of the luminescent device in accordance with the present invention. An anode 2, a hole transport layer 5, an electron transport layer 6 and a cathode 4 are formed on a substrate 1, in that order. The hole transport layer 5 and the electron transport layer 6 function as a luminescent layer 3. In such a configuration, a usable hole transport layer 5 is generally composed of a luminescent material having hole transportability or a mixture including such a material and a non-luminescent material having hole transportability. The luminescent and non-luminescent materials may also have electron transportability. The electron transport layer 6 may be composed of a luminescent material having electron transportability or a mixture including such a material and a non-luminescent material having electron transportability. The luminescent and non-luminescent materials may also have hole transportability.

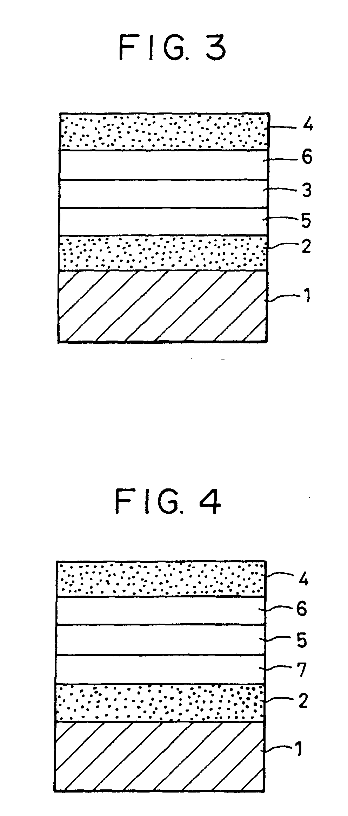

Fig. 3 is a schematic cross-sectional view of a further embodiment of the luminescent device in accordance with the present invention. An anode 2, a hole transport layer 5, a luminescent layer 3, an electron transport layer 6 and a cathode 4 are formed on a substrate 1 in that order. In this configuration, carrier transport and luminescence are performed in the individual layers. Such a configuration permits a wide variety of combinations of a material having excellent hole transportability, a material having excellent electron transportability and a material having excellent luminescence. Further, the configuration permits the use of various compounds emitting light at different wavelengths; hence the hue of the luminescent light can be controlled over a wide range. Effective trap of holes and electrons (or excimers) in the central luminescent layer will increase the luminescent efficiency.

Fig. 4 is a cross-sectional view of another luminescent device in accordance with the present invention. An anode 2, a hole injection-transport layer 7, a hole transport layer 5, an electron transport layer 6, and a cathode 4 are formed on a substrate 1, in that order. The hole injection-transport layer 7 facilitates hole injection from the anode 2. Thus, the luminescent device can maintain high efficiency for long driving times. In such a configuration, the hole transport layer 5 and/or the electron transport layer 6 function as a luminescent layer.

The compounds represented by the general formulae [1] and [2] have significantly superior luminescent characteristics to conventional compounds and can be used in all the electric field luminescent devices shown in Figs. 1 to 4.

The compounds represented by the general formulae [1] and [2] have hole transportability and/or electron transportability depending on the structures thereof. In all the embodiments shown in Figs. 1 to 4, the compounds represented by the general formula [1] may be used alone or in combination, and the compounds represented by the general formula [2] may also be used alone or in combination. Alternatively, the compounds represented by the general formulae [1] and [2] may be used in combination.

As components of the luminescent layer in the luminescent device in accordance with the present invention, hole transport materials studied in the field of electrophotographic photosensitive members and known luminescent hole transport compounds as shown in Tables 1 to 5 or electron transport compounds and known luminescent electron transport materials as shown in Table 6 to 9 can be used with the compounds represented by the general formulae [1] and [2]. These compounds are used alone or in combination.

Table 10 illustrates examples of dopant dyes. The addition of a trace amount of dopant dye in the luminescent layer will significantly increase the luminescent efficiency or will change the luminescent color.

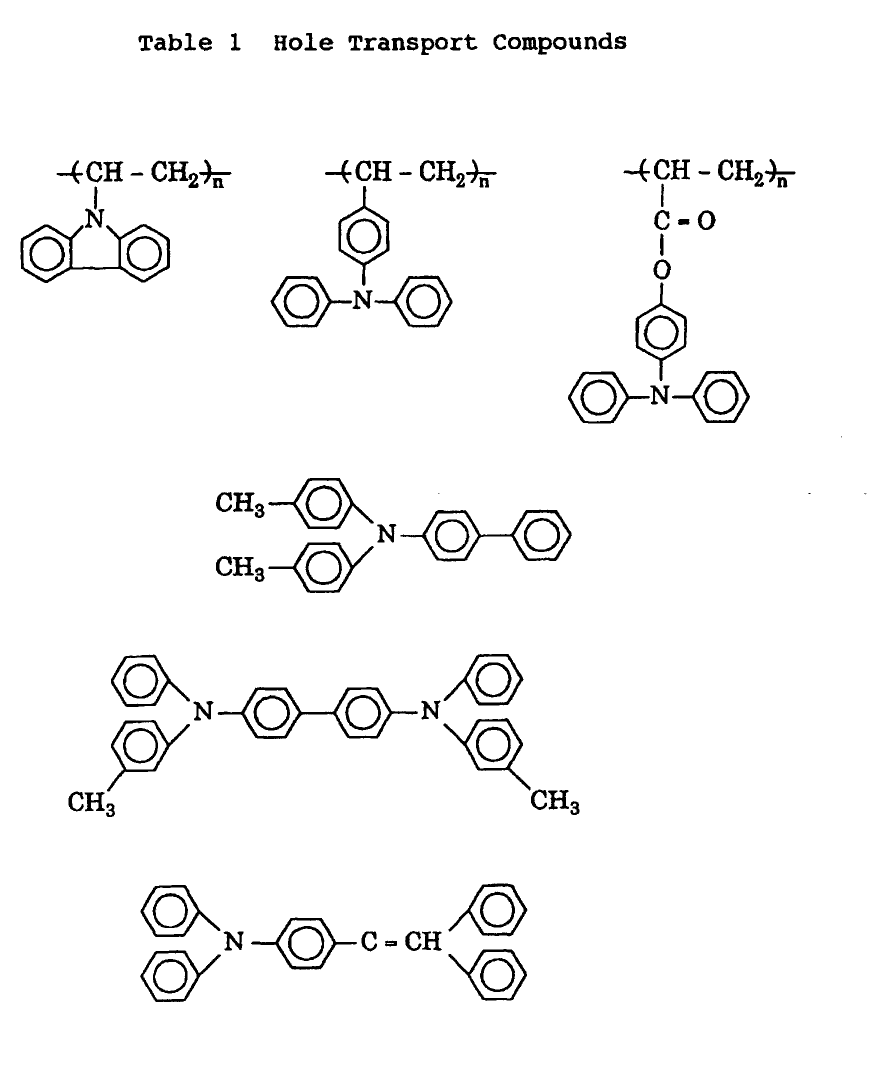

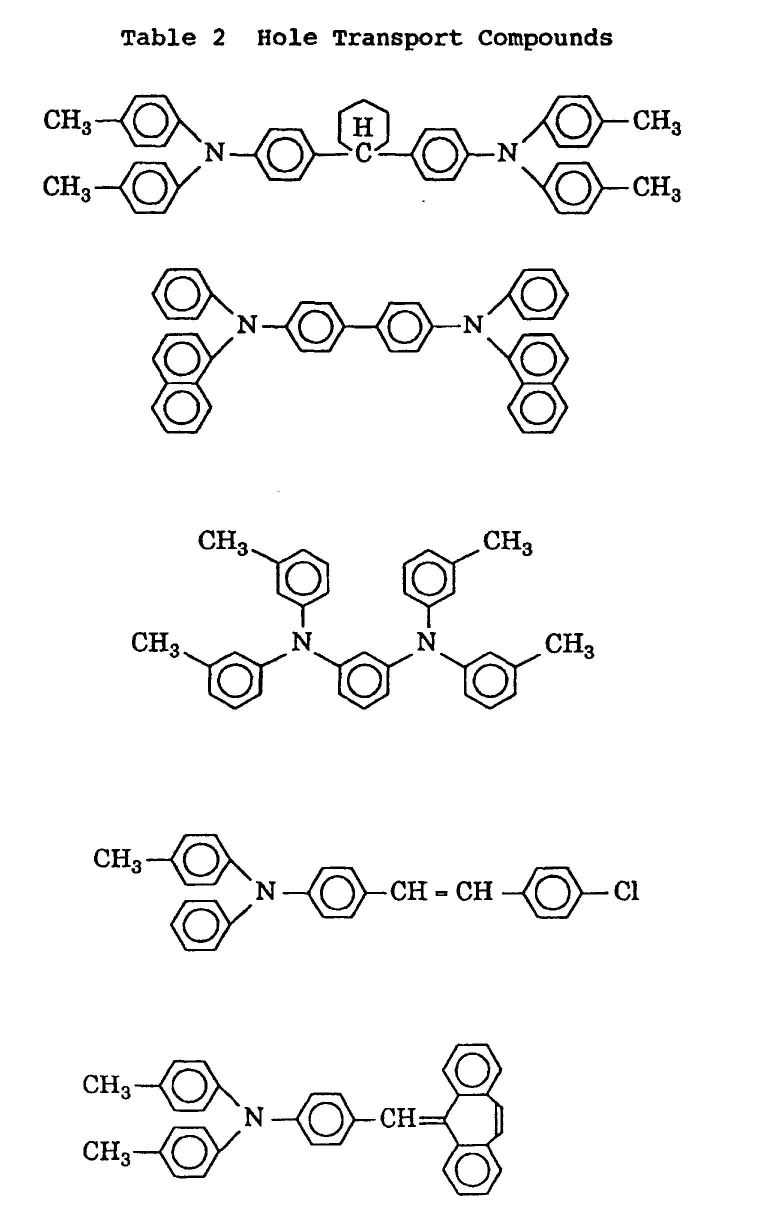

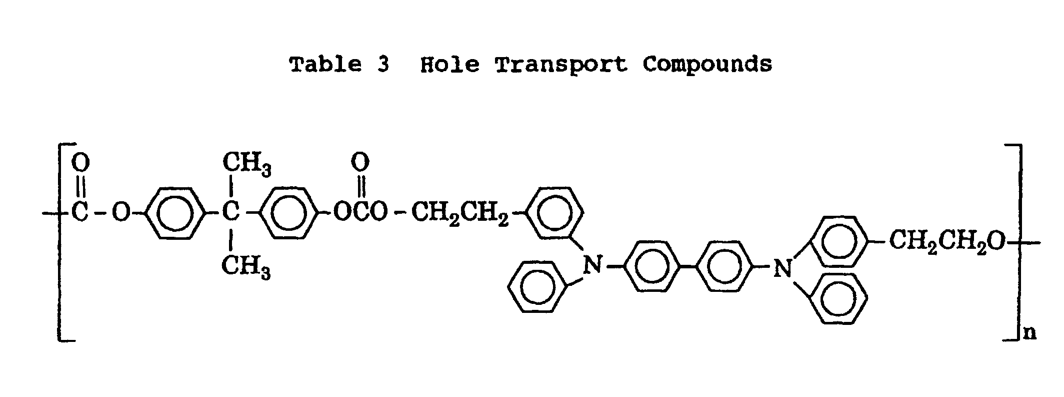

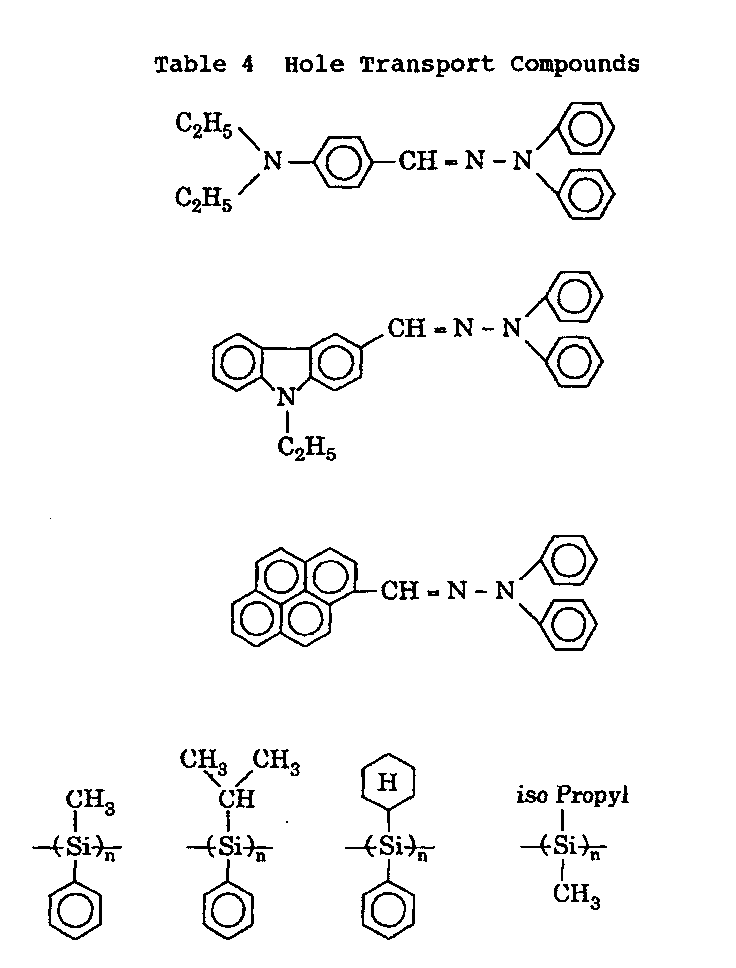

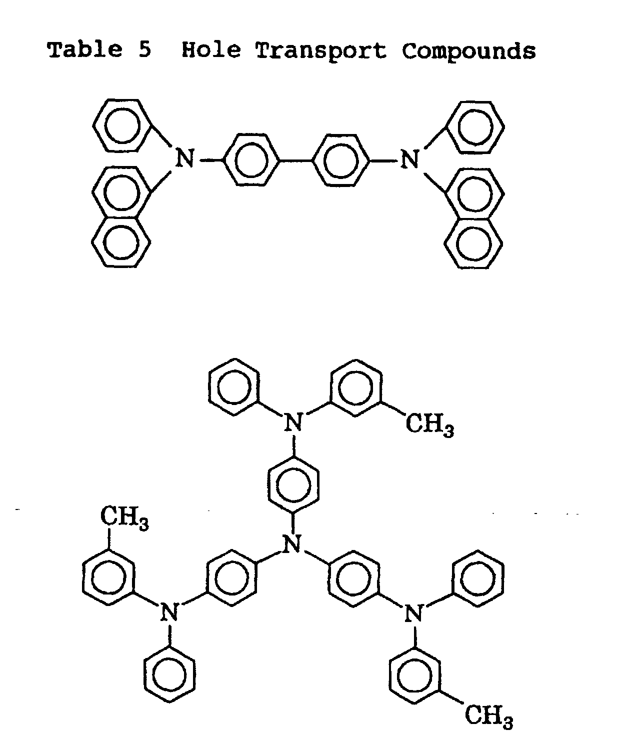

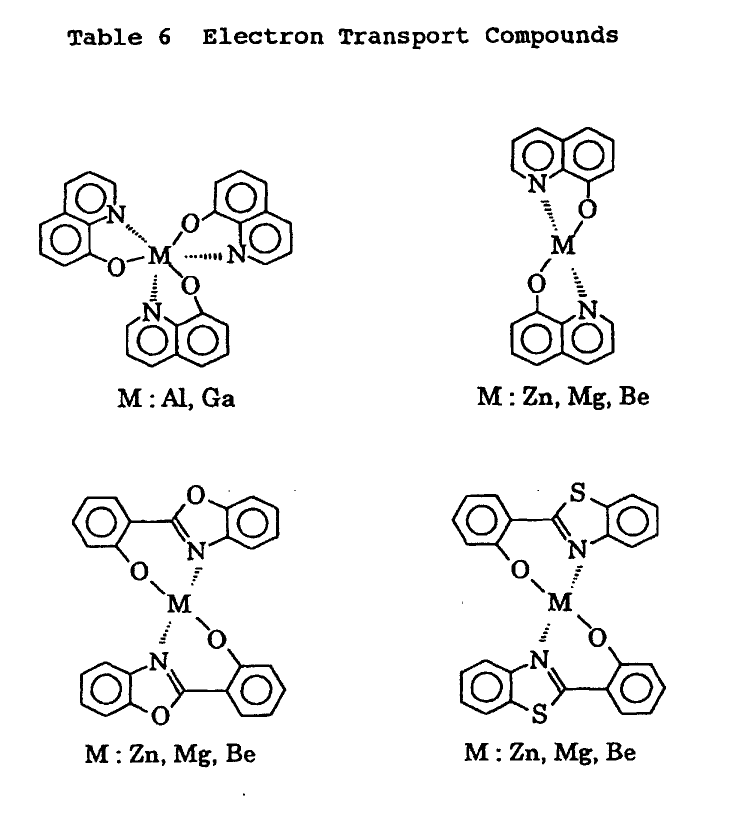

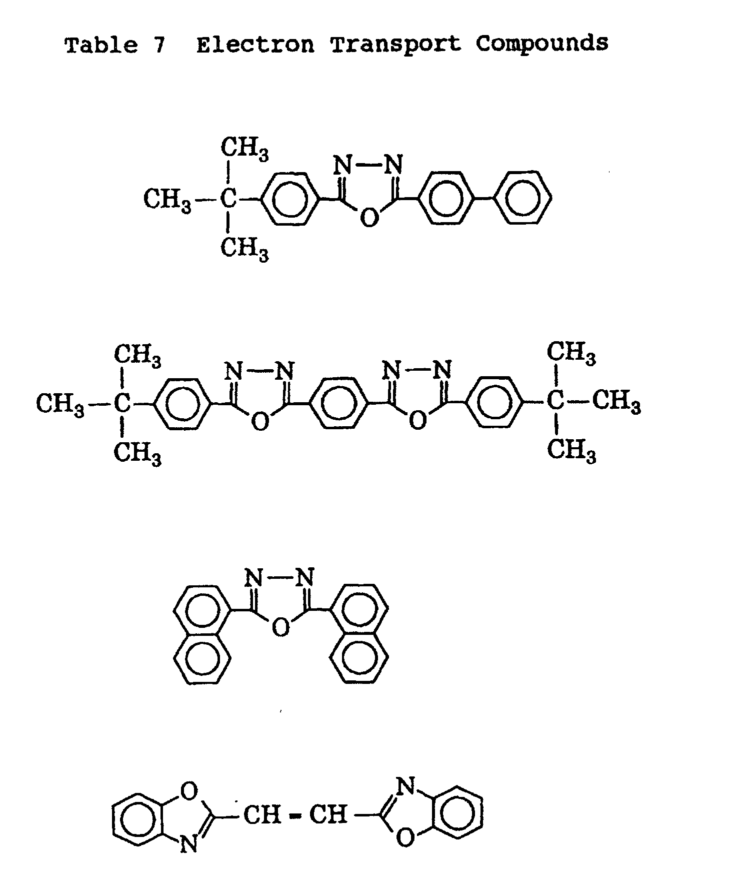

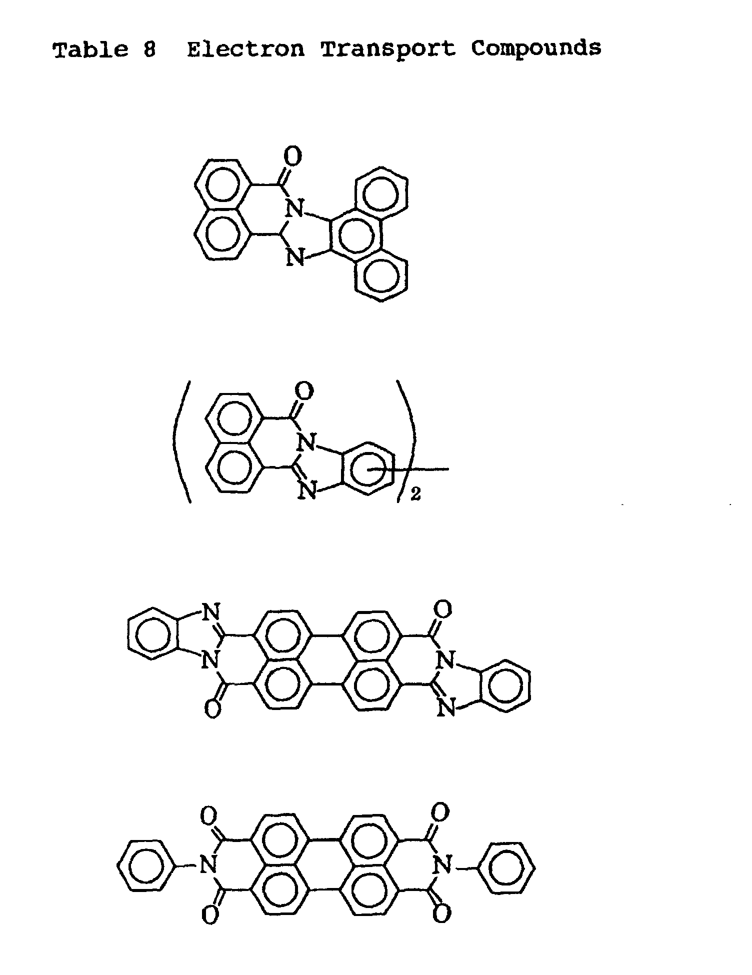

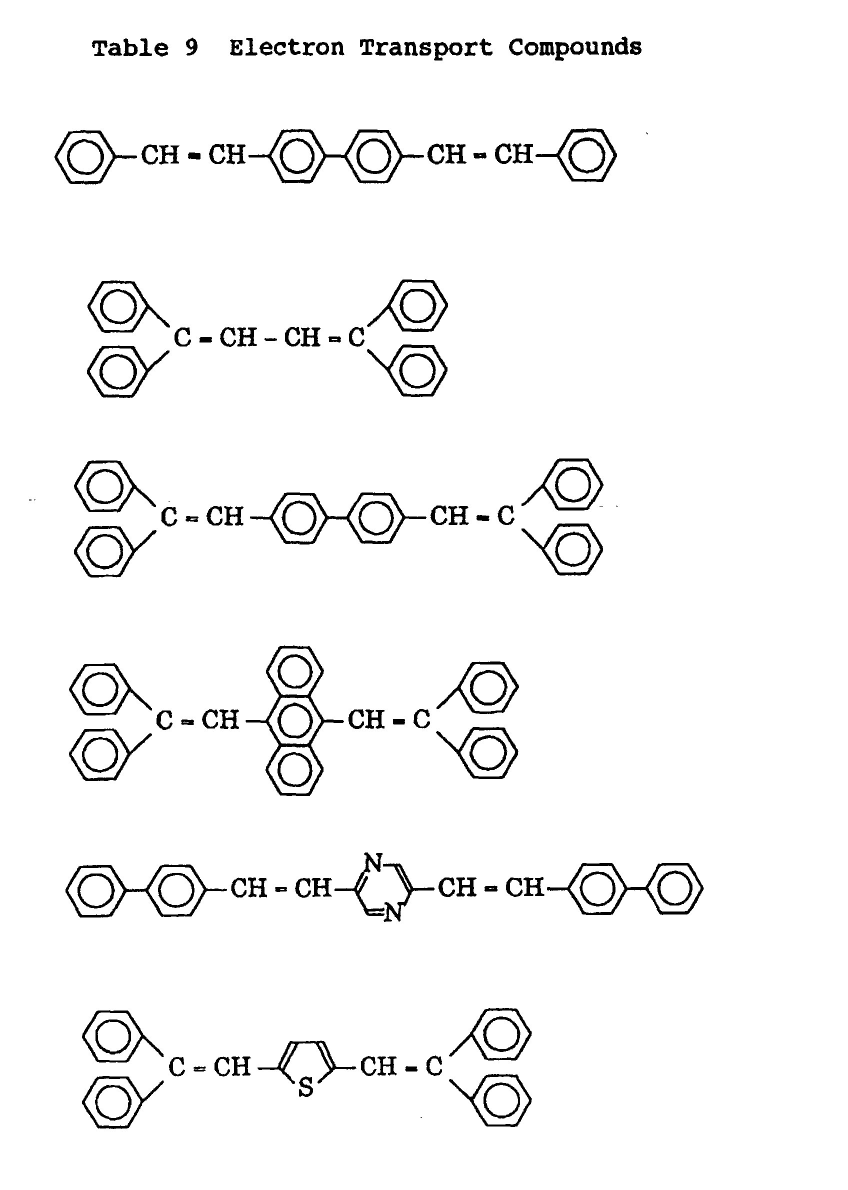

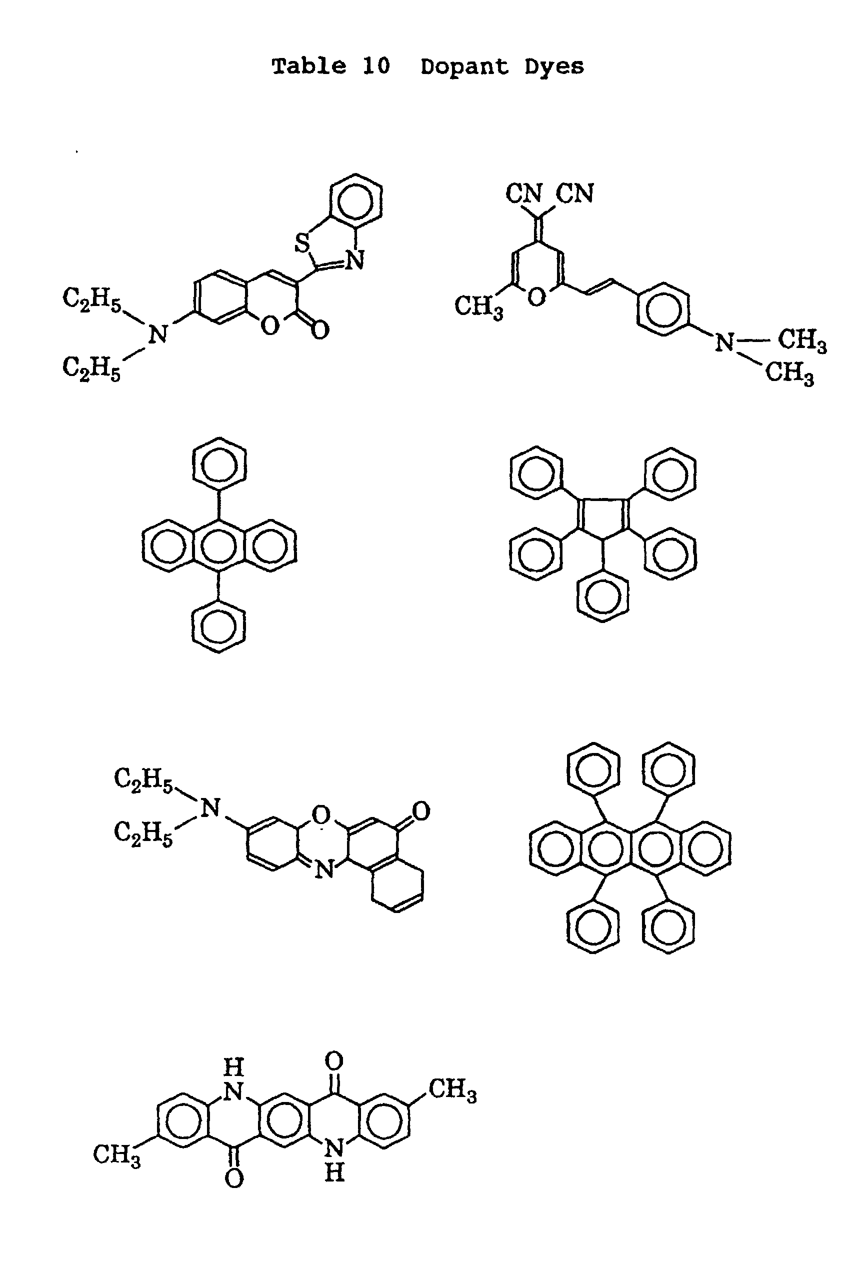

In the luminescent device in accordance with the present invention, the luminescent layer containing the compounds represented by the general formulae [1] and [2] and, if present, the other organic layer are generally formed by a vacuum deposition process or using a binding resin.

Non-limiting examples of the binding resins include polyvinyl carbazole resins, polycarbonate resins, polyester resins, polyarylate resins, butyral resins, polystyrene resins, polyvinyl acetal resins, diallyl phthalate resins, acrylic resins, methacrylic resins, phenol resins, epoxy resins, silicon resins, polysulfone resins, and urea resins. These binding resins can be used alone or in combination.

Preferable anode materials have large work functions. Examples of such materials include nickel, gold, platinum, palladium, selenium, rhenium, and iridium; alloys thereof; and tin oxide, indium tin oxide, and copper iodide. Conductive polymers, such as poly(3-methylthiophene), polyphenylene sulfide and polypyrrole are also usable.

In contrast, preferable cathode materials have small work functions. Examples of such materials include silver, lead, tin, magnesium, aluminum, calcium, manganese, indium and chromium, and alloys thereof.

It is preferable that at least one electrode of the anode and cathode transmits 50% or more of incident light over the wavelength region of the luminescent light.

As the transparent substrate, glass and plastic films are used in the present invention.

### [Examples]

The present invention is described in further detail with reference to the following examples.

### Synthesis of N,N,N',N'-tetra-(1-naphthyl)-2,7-diamino-9,9-dimethylfluorene (Compound 13)

Into a 100-ml egg-plant type flask, 2.24 g (10 mmol) of 2,7-diamino-9,9-dimethylfluorene, 15.22 g (160 mmol) of 1-iodonaphthalene, 6.91 g(50 mmol) of potassium carbonate, 12.71 g (200 mmol) of powdered copper, and 50 ml of o-dichlorobenzene were fed, and the mixture was refluxed with stirring for 24 hours.

The reactant solution was cooled and then filtered, and the filtrate was concentrated under reduced pressure. Into the concentrated solution, 35 ml of acetone was added and then filtered to collect precipitated crude crystal. The crude crystal was purified through a silica gel column using a toluene-hexane mixture, and 6.13 g (yield: 84.1%) of pale yellow fine crystal N,N,N',N'-tetra-(1-naphthyl)-2,7-diamino-9,9-dimethylfluorene (Compound 13) was prepared.

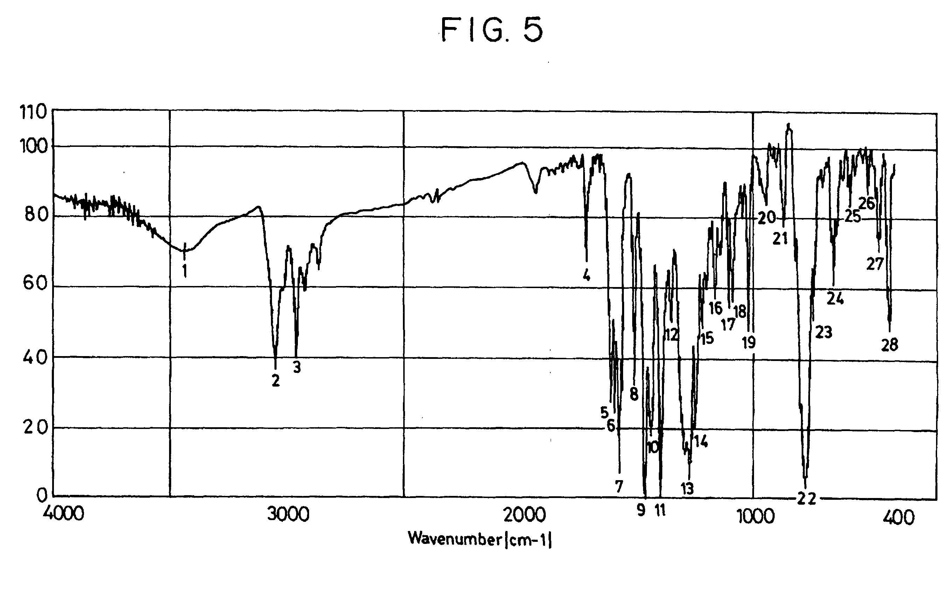

The melting point (Tm) and the glass transition temperature (Tg) of the resulting compound were 331.0 to 332.7°C and 169°C, respectively, according to differential scanning calorimetry using Pyris 1 by Perkin Elmer Corporation. Fig. 5 is an IR spectrum of the compound by a KBr tablet method using an FT-IR spectrophotometer (FT-IR-420) by JASCO.

### EXAMPLE 1

An indium tin oxide (ITO) film with a thickness of 100 nm was formed on a glass substrate by a sputtering process. After the transparent substrate was cleaned, a layer of Compound 12 with a thickness of 65 nm was deposited thereon at a deposition rate of 0.2 to 0.3 nm/sec, and then a Mg-Ag metallic electrode having an atomic ratio of Mg:Ag = 10:1 was formed by a vacuum deposition process at a deposition rate of 2.0 nm/sec under a vacuum pressure of 3 to 4×10⁻⁶ torr. A luminescent device was thereby formed.

A direct current of 10 V was applied between the ITO anode and the Mg-Ag cathode of the luminescent device. A current flow of 175 mA/cm² and a green luminescence with a luminance of 5,300 cd/m² were observed. A voltage with a current density of 3.0 mA/cm² was applied to the sample for 100 hours. The luminance was 160 cd/m² at the start and changed to 140 cd/m² at the end.

### EXAMPLES 2 TO 6

Luminescent devices were prepared as in EXAMPLE 1 using Compounds 21, 36, 47, 72 and 88 instead of Compound 12. Table 11 shows the characteristics of these luminescent devices.

**Table 11**

| EXAMPLE | Compound | Initial | | After 100 hours | |
|---|---|---|---|---|---|
| | | Applied Voltage (V) | Luminance (cd/m²) | Applied Voltage (V) | Luminance (cd/m²) |
| 2 | 21 | 5.3 | 350 | 5.9 | 345 |
| 3 | 36 | 6.7 | 275 | 7.8 | 280 |
| 4 | 47 | 4.8 | 345 | 5.7 | 330 |
| 5 | 72 | 4.9 | 550 | 5.5 | 530 |
| 6 | 88 | 5.7 | 450 | 3.8 | 450 |

### COMPARATIVE EXAMPLE 1

A luminescent device was prepared as in EXAMPLE 1 using the following compound instead of Compound 12.

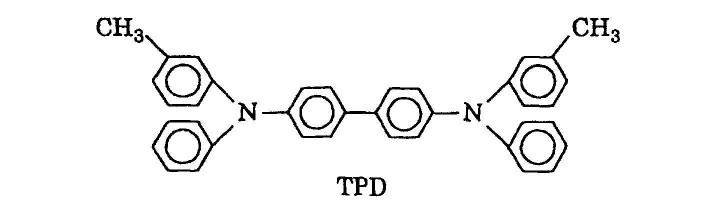

A direct current of 15 V was applied between the ITO anode and the Mg-Ag cathode of the luminescent device. A current flow of 15 mA/cm² and a green luminescence with a luminance of 35 cd/m² were observed. A voltage with a current density of 27 mA/cm² was applied to the sample for 100 hours. The luminance was 100 cd/m² at the start and decreased to 8 cd/m² at the end.

The results of EXAMPLES 1 to 6 and COMPARATIVE EXAMPLE 1 show that the compounds in accordance with the present invention have high luminance and prolonged life compared to the comparative amine compound.

### EXAMPLE 7

A luminescent device shown in Fig. 4 was prepared as follows. An indium tin oxide (ITO) anode 2 with a thickness of 100 nm was formed on a glass substrate by a sputtering process. After the transparent substrate was cleaned, a m-MTDATA hole injection-transport layer 7 with a thickness of 20 nm was formed thereon, and a layer of Compound 32 with a thickness of 50 nm was deposited thereon as a hole transport layer 5. Furthermore, an electron transport layer of an electron transport compound (Alq₃) with a thickness of 65 nm was formed thereon, and then an aluminum cathode 4 with a thickness of 140 nm was formed thereon.

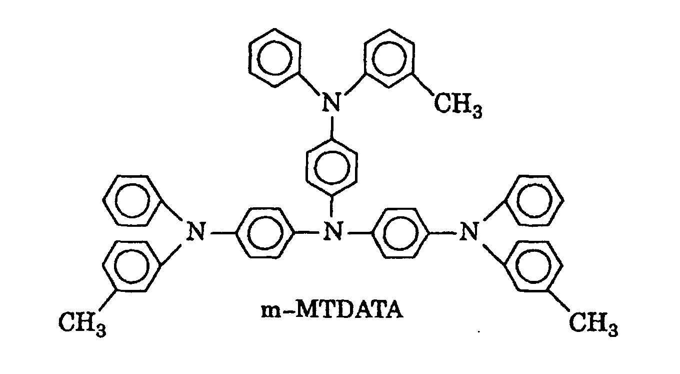

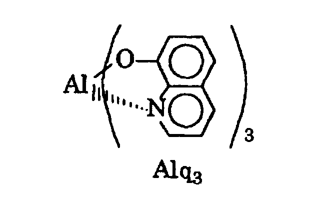

A direct current of 5 V was applied between the ITO anode and the aluminum cathode of the luminescent device. A current flow of 10 mA/cm² and a green luminescence with a luminance of 576 cd/m² were observed. A voltage with a current density of 3.0 mA/cm² was applied to the sample for 100 hours. The luminance was 265 cd/m² at the start and slightly changed to 250 cd/m² at the end.

As described above, luminescent devices using compounds represented by the general formulae [1] and [2] in accordance with the present invention have significantly high luminance for a low applied-voltage, and high durability. A large device can be readily formed by a vacuum deposition process or a casting process with relatively low production costs.

While the present invention has been described with reference to what are presently considered to be the preferred embodiments, it is to be understood that the invention is not limited to the disclosed embodiments. On the contrary, the invention is intended to cover various modifications and equivalent arrangements included within the scope of the appended claims. The scope of the following claims is to be accorded the broadest interpretation so as to encompass all such modifications and equivalent structures and functions.

## Claims

1. A triarylamine compound represented by the following general formula [1]: 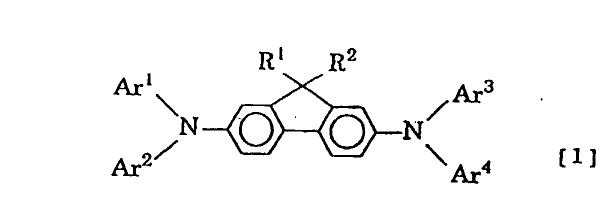 wherein:
R¹ and R² are each independently a hydrogen atom, a halogen atom, a substituted or unsubstituted alkyl group, a substituted or unsubstituted alkoxy group, or a substituted or unsubstituted aryl group,
Ar¹, Ar², Ar³ and Ar⁴ are each a substituted or unsubstituted aryl or heterocyclic group which may be the same or different from each other selected from phenyl, biphenyl, terphenyl, naphthyl, anthryl, phenanthryl, pyridyl, furyl, thienyl and carbazolyl;
and at least one of Ar¹, Ar², Ar³, and Ar⁴ is a fused aromatic ring selected from naphthyl, anthryl, acenaphthyl, phenanthryl, naphthathenyl and fluoranthenyl.

2. A triarylamine compound according to claim 1, wherein at least one of Ar¹, Ar², Ar³ and Ar⁴ is selected from a naphthyl group and an anthryl group.

3. A triarylamine compound according to claim 1 or claim 2 wherein the substituents R¹, R², Ar¹, Ar², Ar³ and Ar⁴ have the formulae indicated in the following table:

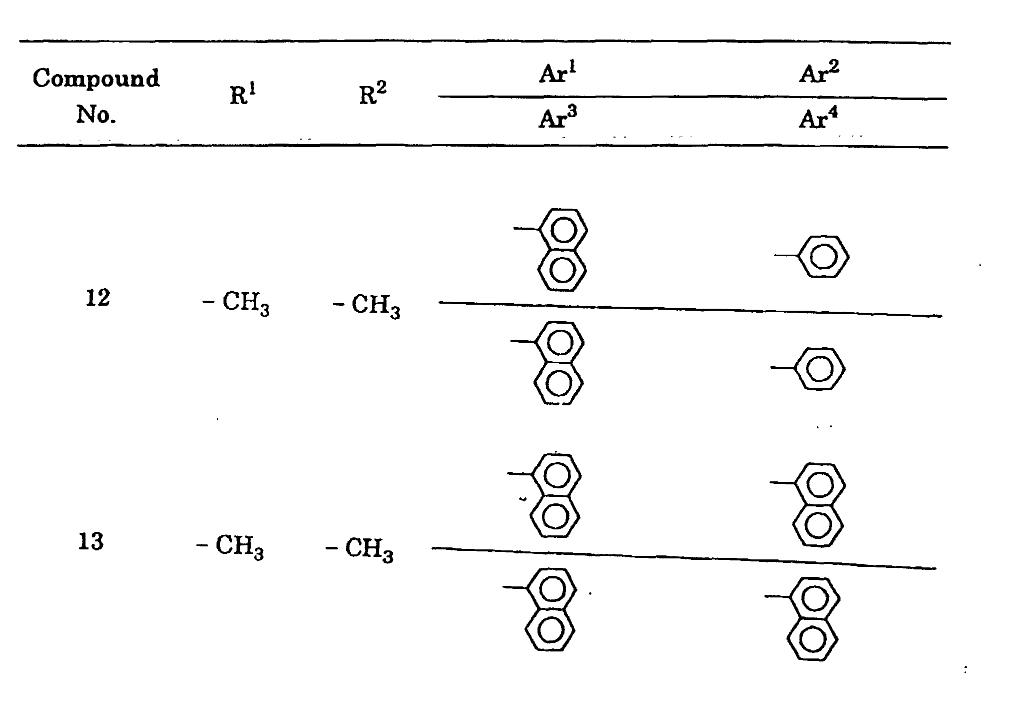

4. A triarylamine compound represented by the following general formula[1]: 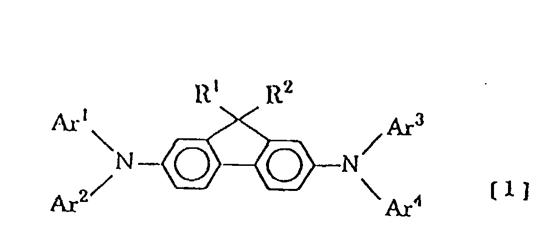 wherein the substituents R¹, R², Ar¹, Ar², Ar³ and Ar⁴ have the formulae indicated in the following table:

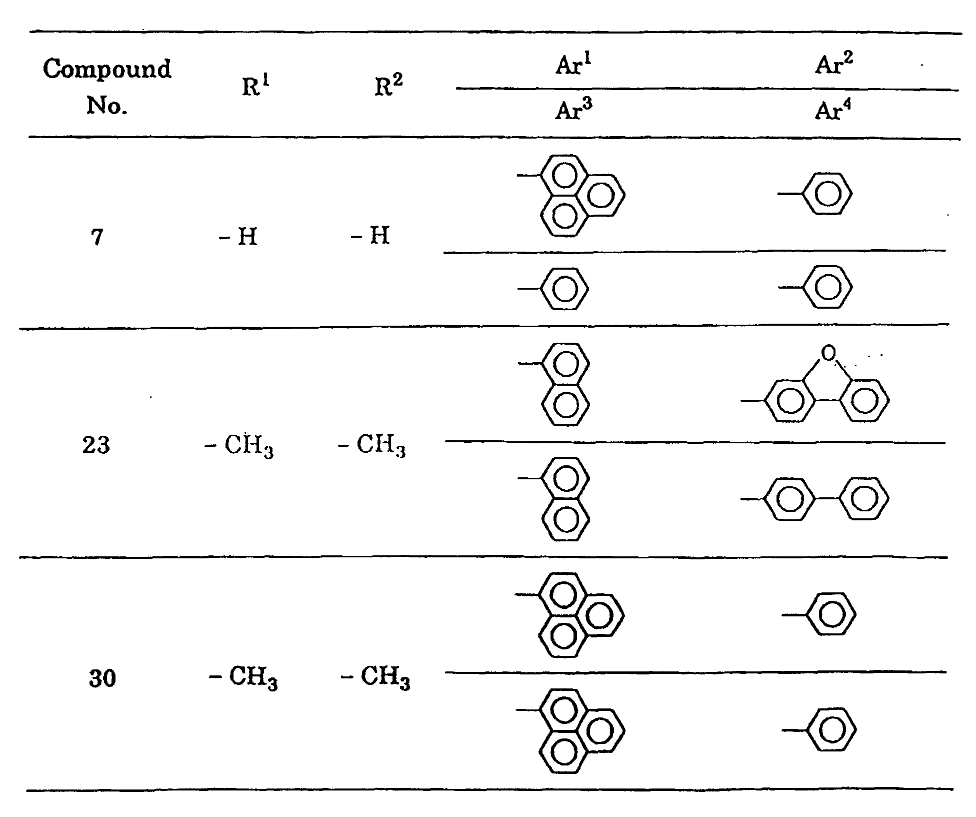

5. A triarylamine compound represented by the following general formula [2]: 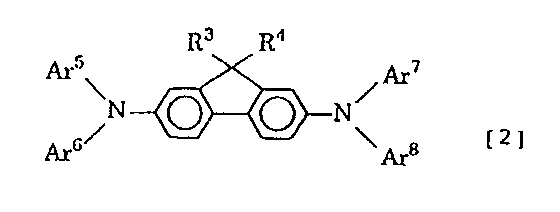 wherein:
R³ and R⁴ are each independently a hydrogen atom, a halogen atom, a substituted or unsubstituted alkyl group, a substituted or unsubstituted alkoxy group, or a substituted or unsubstituted aryl group;
Ar⁵, Ar⁶, Ar⁷ and Ar⁸ are each a substituted or unsubstituted aryl or heterocyclic group, which may be the same or different from each other selected from phenyl, biphenyl, terphenyl, naphthyl, anthryl, penanthryl, pyridyl, furyl, thienyl and carbazolyl;
and at least one of Ar⁵, Ar⁶, Ar⁷ and Ar⁸ is a π-conjugated aromatic hydrocarbon having 12 or more carbon atoms selected from polyphenyls and stilbene derivatives.

6. A triarylamine compound according to claim 5, wherein at least one of Ar⁵, Ar⁶, Ar⁷ and Ar⁸ is selected from biphenyl, m-terphenyl, p-terphenyl, quaterphenyl, styryl and phenylstyryl.

7. A triarylamine compound represented by the following general formula [2]: 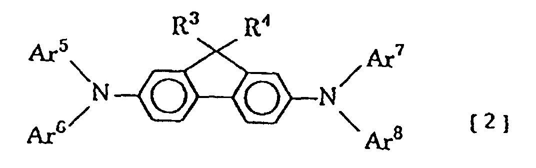 wherein the substituents R³, R⁴, Ar⁶, Ar⁷ and Ar⁸ have the formulae indicated in the following table:

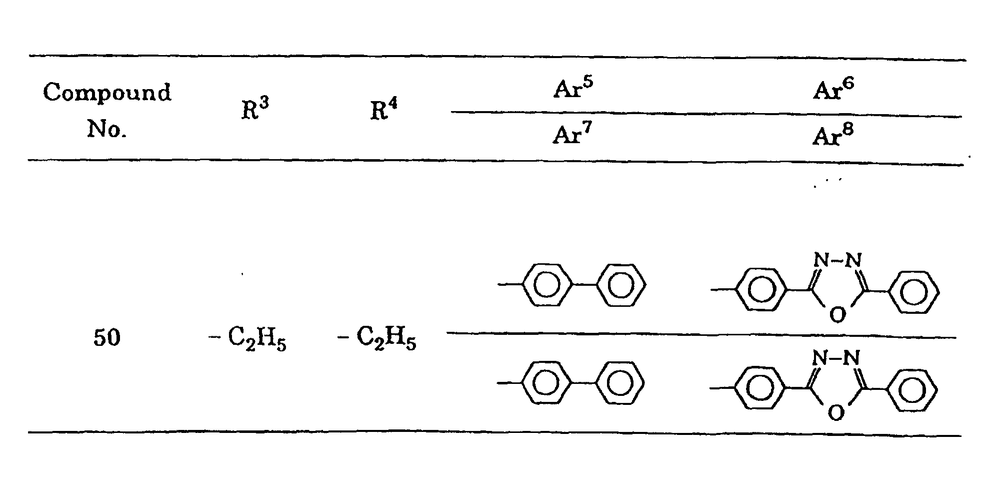

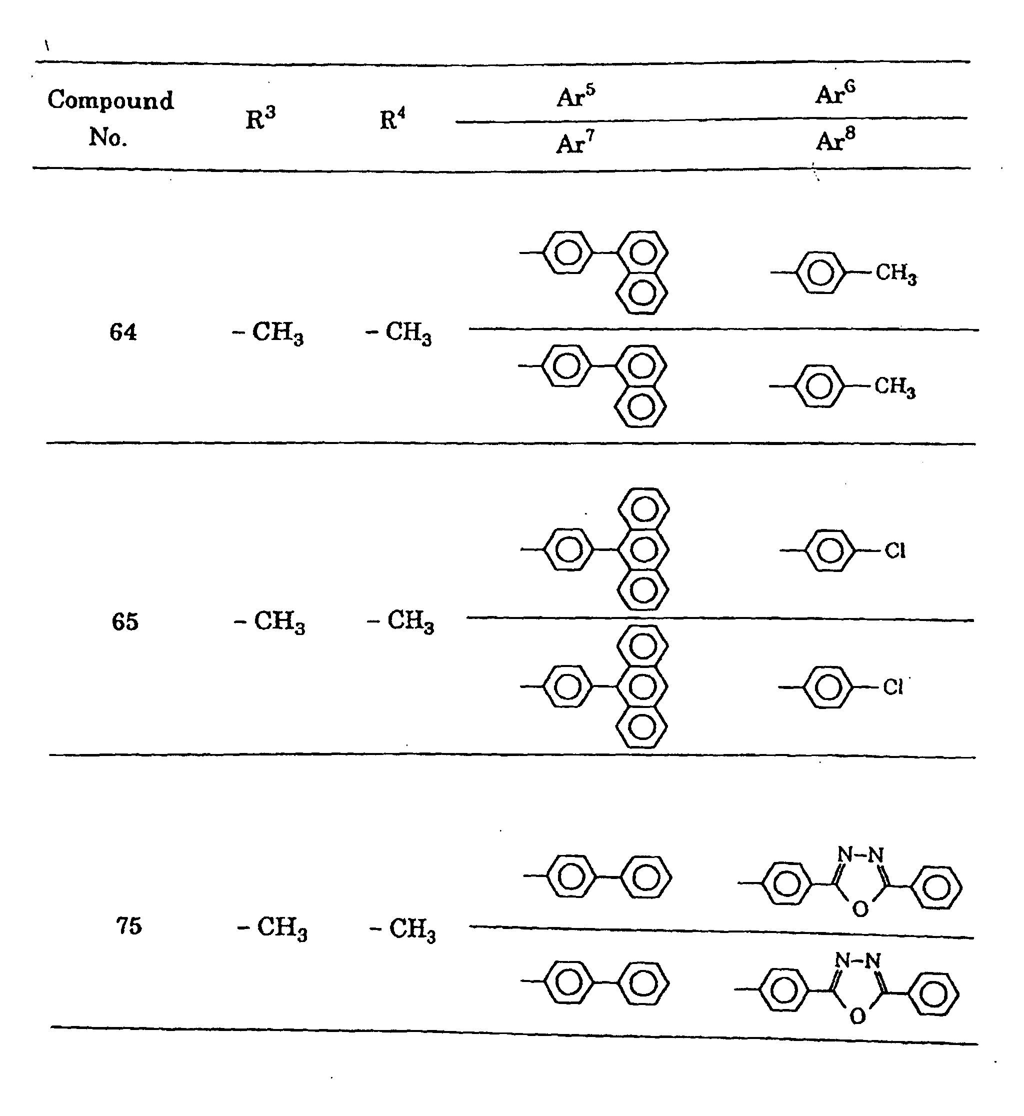

8. A luminescent device comprising a pair of electrodes and an organic compound layer disposed therebetween, the organic compound layer comprising a compound as claimed in any preceding claim.

9. A luminescent device according to claim 8, wherein the thickness of the organic compound layer is less than 2 µm.

10. A luminescent device according to claim 9, wherein the thickness of the organic compound layer is in a range of 0.05 to 0.5 µm.

11. A luminescent device according to claim 8, wherein the organic compound later is used as a hole transport layer or a luminescent layer.

12. A luminescent device comprising a pair of electrodes and an organic compound layer disposed therebetween, the organic compound layer comprising a compound as claimed in any one of claims 1 to 4 and a compound as claimed in any one of claims 5 to 7.

## Patentansprüche

1. Triarylaminverbindung, dargestellt durch die folgende allgemeine Formel [1]: 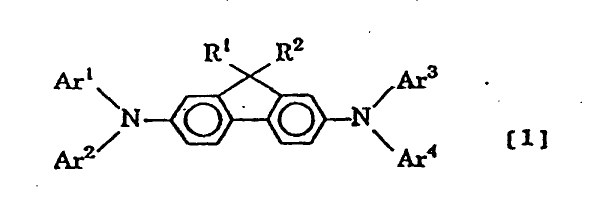 in der:
R¹ und R² unabhängig voneinander ein Wasserstoffatom, ein Halogenatom, eine substituierte oder unsubstituierte Alkylgruppe, eine substituierte oder unsubstituierte Alkoxygruppe, oder eine substituierte oder unsubstituierte Arylgruppe sind,
Ar¹, Ar², Ar³ und Ar⁴ jeweils eine substituierte oder unsubstituierte Arylgruppe oder heterocyclische Gruppe sind, welche dieselbe oder unterschiedlich voneinander sein können, gewählt aus Phenyl, Biphenyl, Terphenyl, Naphthyl, Anthryl, Phenanthryl, Pyridyl, Furyl, Thienyl und Carbazolyl;
und mindestens eine Gruppe von Ar¹, Ar², Ar³ und Ar⁴ ein kondensierter aromatischer Ring ist, gewählt aus Naphthyl, Anthryl, Acenaphthyl, Phenanthryl, Naphthathenyl und Fluoranthenyl.

2. Triarylaminverbindung nach Anspruch 1, wobei mindestens eine Gruppe von Ar¹, Ar², Ar³ und Ar⁴ aus einer Naphthylgruppe und einer Anthrylgruppe gewählt ist.

3. Triarylaminverbindung nach Anspruch 1 oder Anspruch 2, wobei die Substituenten R¹, R², Ar¹, Ar², Ar³ und Ar⁴ die in der folgenden Tabelle angegebenen Formeln haben:

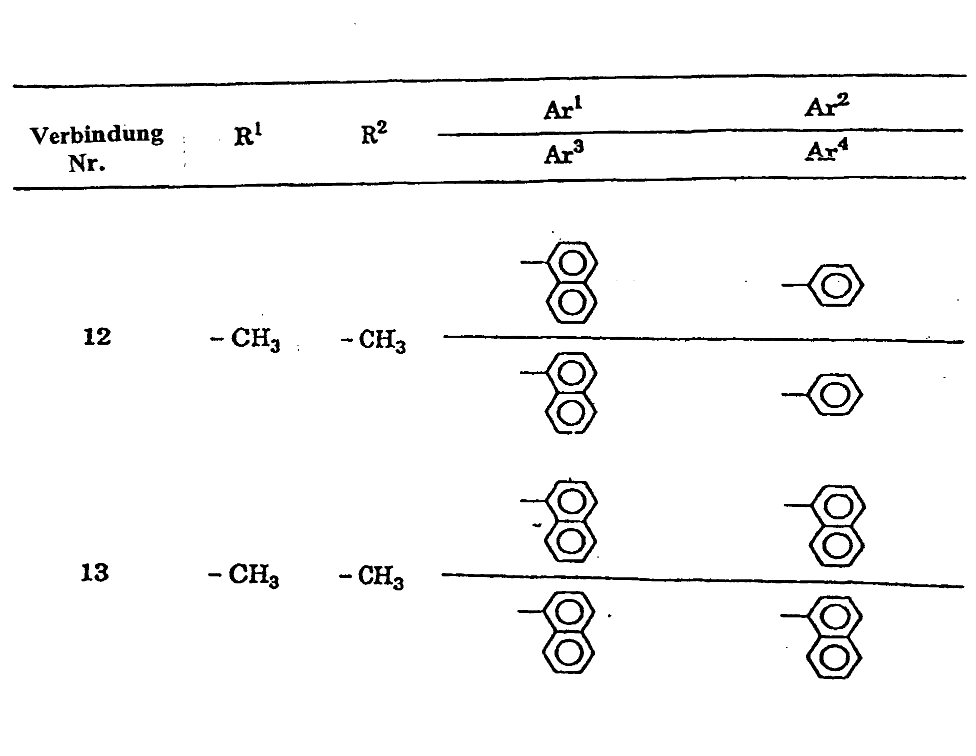

4. Triarylaminverbindung, dargestellt durch die folgende allgemeine Formel [1]: 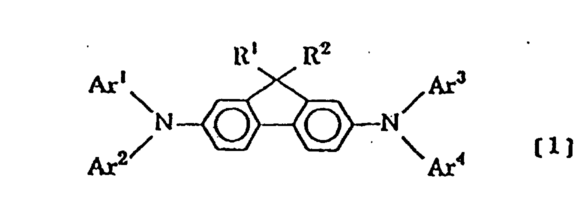 in der die Substituenten R¹, R², Ar¹, Ar², Ar³ und Ar⁴ die in der folgenden Tabelle angegebenen Formeln haben:

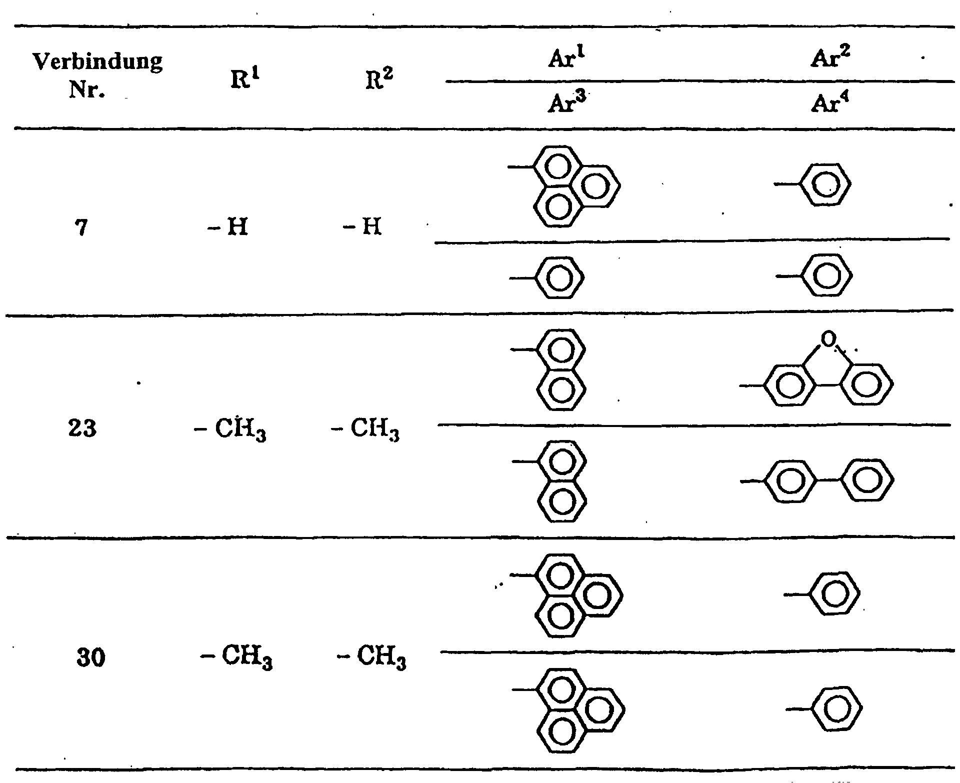

5. Triarylaminverbindung, dargestellt durch die folgende allgemeine Formel [2]: 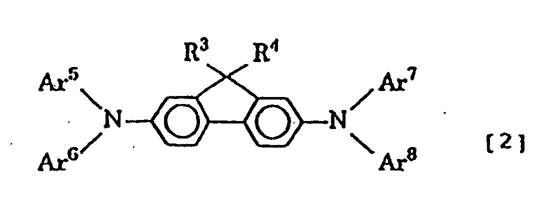 in der:
R³ und R⁴ unabhängig voneinander ein Wasserstoffatom, ein Halogenatom, eine substituierte oder unsubstituierte Alkylgruppe, eine substituierte oder unsubstituierte Alkoxygruppe, oder eine substituierte oder unsubstituierte Arylgruppe sind;
Ar⁵, Ar⁶, Ar⁷ und Ar⁸ jeweils eine substituierte oder unsubstituierte Arylgruppe oder heterocyclische Gruppe sind, welche dieselbe oder unterschiedlich voneinander sein können, gewählt aus Phenyl, Biphenyl, Terphenyl, Naphthyl, Anthryl, Phenanthryl, Pyridyl, Furyl, Thienyl und Carbazolyl;
und mindestens eine Gruppe von Ar⁵, Ar⁶, Ar⁷ und Ar⁸ ein π-konjugierter aromatischer Kohlenwasserstoff mit 12 oder mehr Kohlenstoffatomen ist, gewählt aus Polyphenylen und Stilben-Derivaten.

6. Triarylaminverbindung nach Anspruch 5, wobei mindestens eine Gruppe von Ar⁵, Ar⁶, Ar⁷ und Ar⁸ gewählt ist aus Biphenyl, m-Terphenyl, p-Terphenyl, Quaterphenyl, Styryl und Phenylstyryl.

7. Triarylaminverbindung, dargestellt durch die folgende allgemeine Formel [2]: 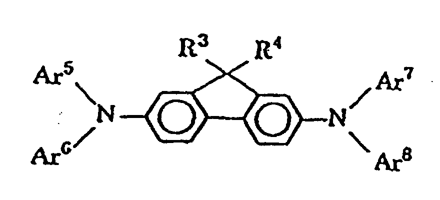 in der die Substituenten R³, R⁴, Ar⁶, Ar⁷ und Ar⁸ die in der folgenden Tabelle angegebenen Formeln haben:

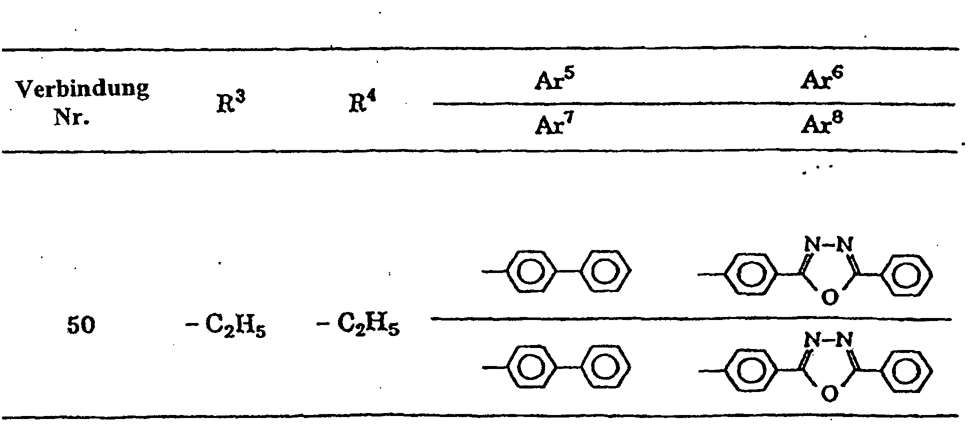

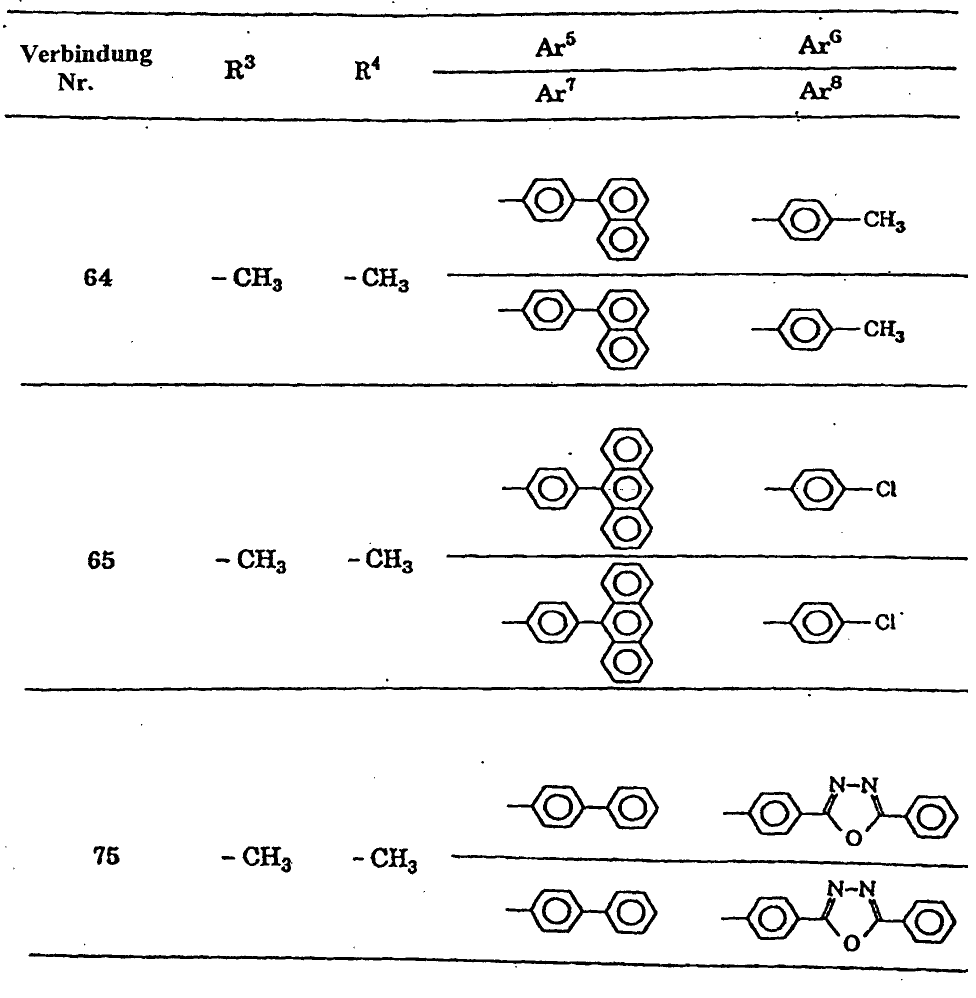

8. Lumineszenzvorrichtung, umfassend ein Elektrodenpaar und eine dazwischen angeordnete organische Verbindungsschicht, wobei die organische Verbindungsschicht eine Verbindung umfasst, die in einem der vohergehenden Ansprüche beansprucht ist.

9. Lumineszenzvorrichtung nach Anspruch 8, wobei die Dicke der organischen Verbindungsschicht weniger als 2 µm beträgt.

10. Lumineszenzvorrichtung nach Anspruch 9, wobei die Dicke der organischen Verbindungsschicht in einem Bereich von 0,05 bis 0,5 µm liegt.

11. Lumineszenzvorrichtung nach Anspruch 8, wobei die organische Verbindungsschicht später verwendet wird als Lochtransportschicht oder Lumineszenzschicht.

12. Lumineszenzvorrichtung, umfassend ein Elektrodenpaar und eine dazwischen angeordnete organische Verbindungsschicht, wobei die organische Verbindungsschicht eine Verbindung umfasst, die in einem der Ansprüche 1 bis 4 beansprucht ist, und eine Verbindung, die in einem der Ansprüche 5 bis 7 beansprucht ist.

## Revendications

1. Dérivé de triarylamine représenté par la formule générale [1] suivante : 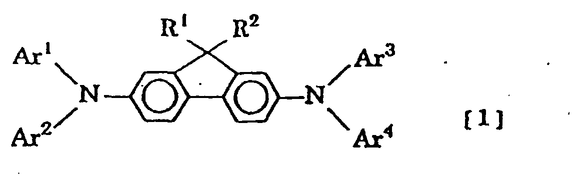 dans laquelle :
R¹ et R² représentent chacun indépendamment un atome d'hydrogène, un atome d'halogène, un groupe alkyle substitué ou non substitué, un groupe alkoxy substitué ou non substitué ou un groupe aryle substitué ou non substitué,
Ar¹, Ar², Ar³ et Ar⁴ représentent chacun un groupe aryle ou hétérocyclique substitué ou non substitué, ces groupes pouvant être identiques ou différents l'un de l'autre, étant choisi entre des groupes phényle, biphényle, terphényle, naphtyle, anthryle, phénanthryle, pyridyle, furyle, thiényle et carbazolyle ;
et au moins un des groupes Ar¹, Ar², Ar³ et Ar⁴ représente un noyau aromatique condensé choisi entre les noyaux naphtyle, anthryle, acénaphtyle, phénanthryle et fluoranthényle.

2. Dérivé de triarylamine suivant la revendication 1, dans lequel au moins un des groupes Ar¹, Ar², Ar³ et Ar⁴ est choisi entre un groupe naphtyle et un groupe anthryle.

3. Dérivé de triarylamine suivant la revendication 1 ou la revendication 2, dans lequel les substituants R¹, R², Ar¹, Ar³ et Ar⁴ répondent aux formules représentées sur le tableau suivant :

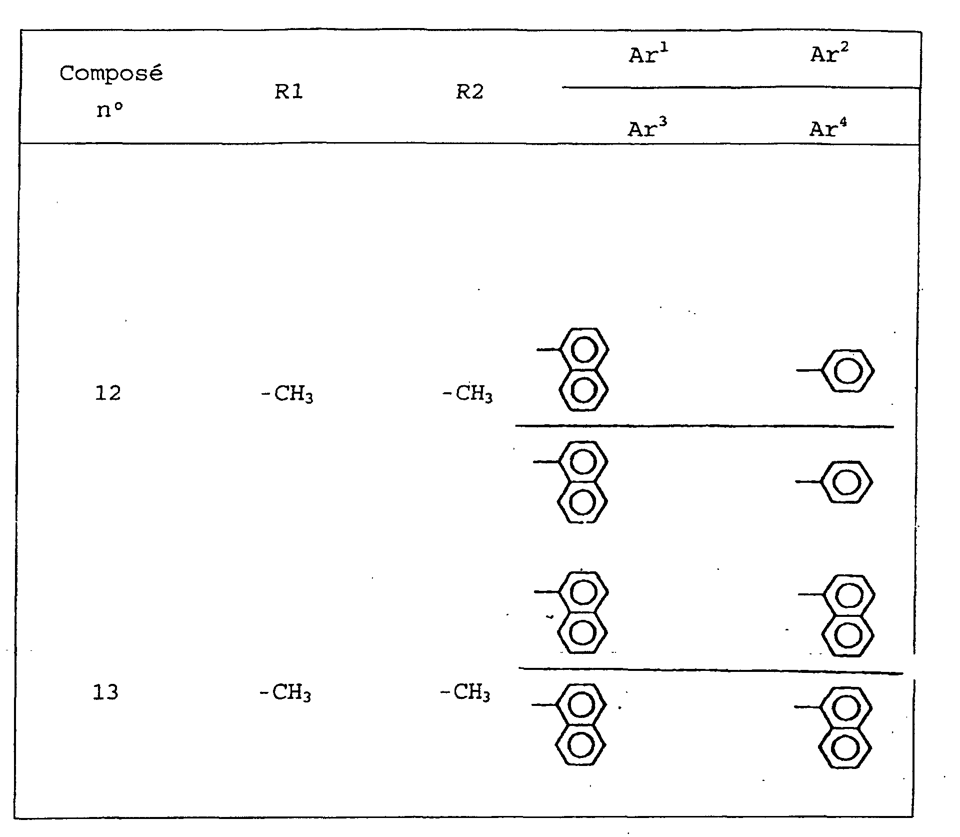

4. Dérivé de triarylamine représenté par la formule générale [1] suivante : 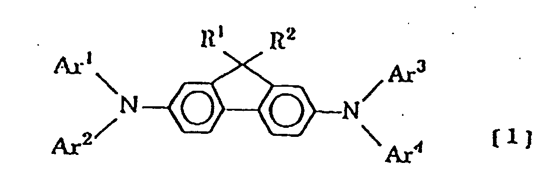 dans laquelle les substituants R¹, R², Ar¹, Ar², Ar³ et Ar⁴ répondent aux formules représentées sur le tableau suivant :

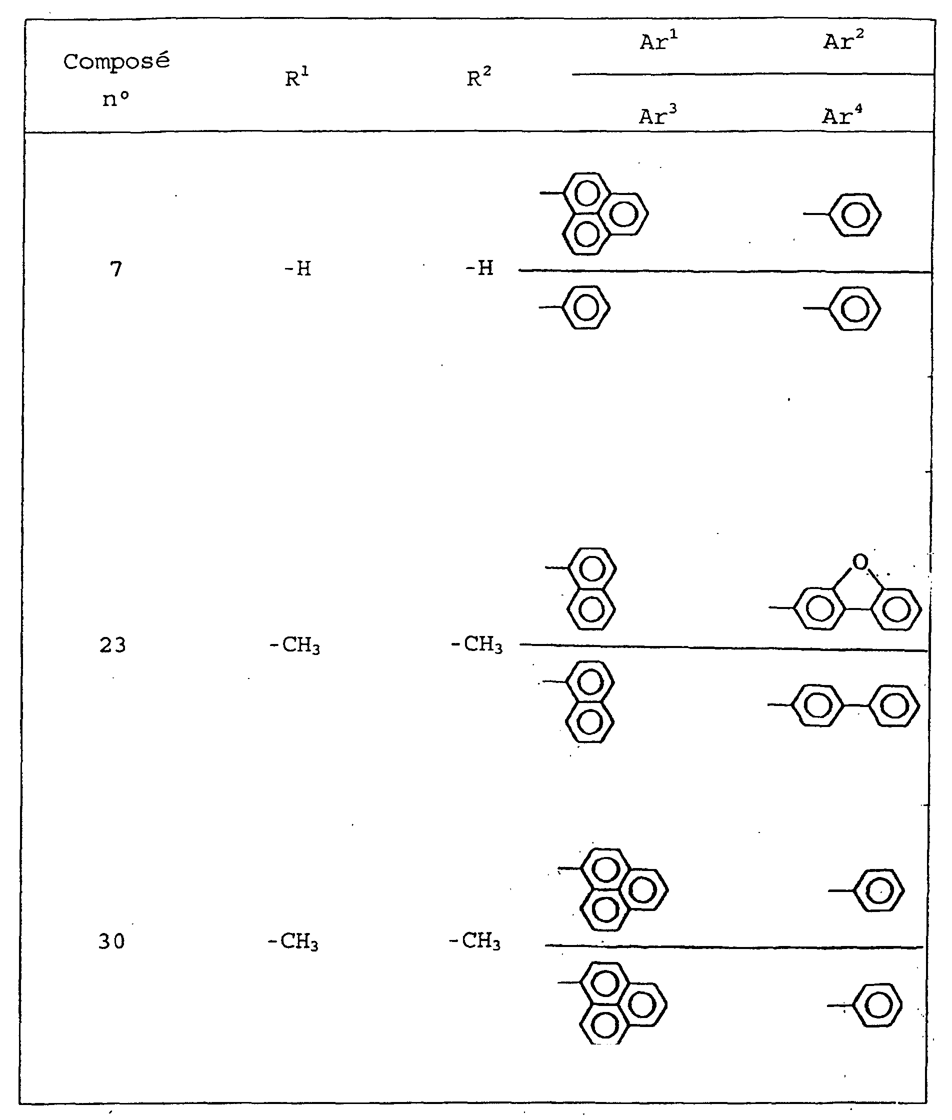

5. Dérivé de triarylamine représenté par la formule générale [2] suivante : 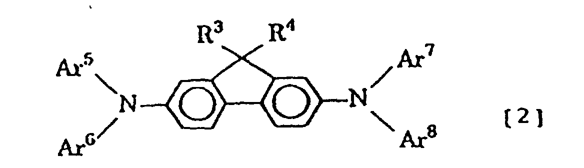 dans laquelle :
R³ et R⁴ représentent chacun indépendamment un atome d'hydrogène, un atome d'halogène, un groupe alkyle substitué ou non substitué, un groupe alkoxy substitué ou non substitué ou un groupe aryle substitué ou non substitué ;
Ar⁵, Ar⁶, Ar⁷ et Ar⁸ représentent chacun un groupe aryle ou hétérocyclique substitué ou non substitué, ces groupes pouvant être identiques ou différents l'un de l'autre, étant choisi entre des groupes phényle, biphénylé, terphényle, naphtyle, anthryle, phénanthryle, pyridyle, furyle, thiényle et carbazolyle ;
et au moins un des groupes Ar⁵, Ar⁶, Ar⁷ et Ar⁸ représente un groupe hydrocarboné aromatique à conjugaison π ayant 12 ou plus de 12 atomes de carbone choisi entre des polyphényles et des dérivés de stilbène.

6. Dérivé de triarylamine suivant la revendication 5, dans lequel au moins un des groupes Ar⁵, Ar⁶, Ar⁷ et Ar⁸ est choisi entre des groupes biphényle, m-terphényle, p-terphényle, quaterphényle, styryle et phénylstyryle.

7. Dérivé de triarylamine représenté par la formule générale [2] suivante : 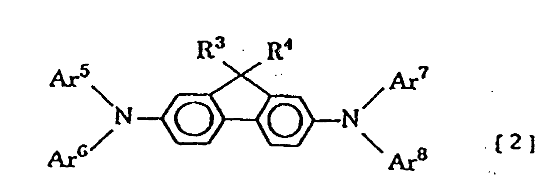 dans laquelle les substituants R³, R⁴, Ar⁶, Ar⁷ et Ar⁸ répondent aux formules représentées sur le tableau suivant :

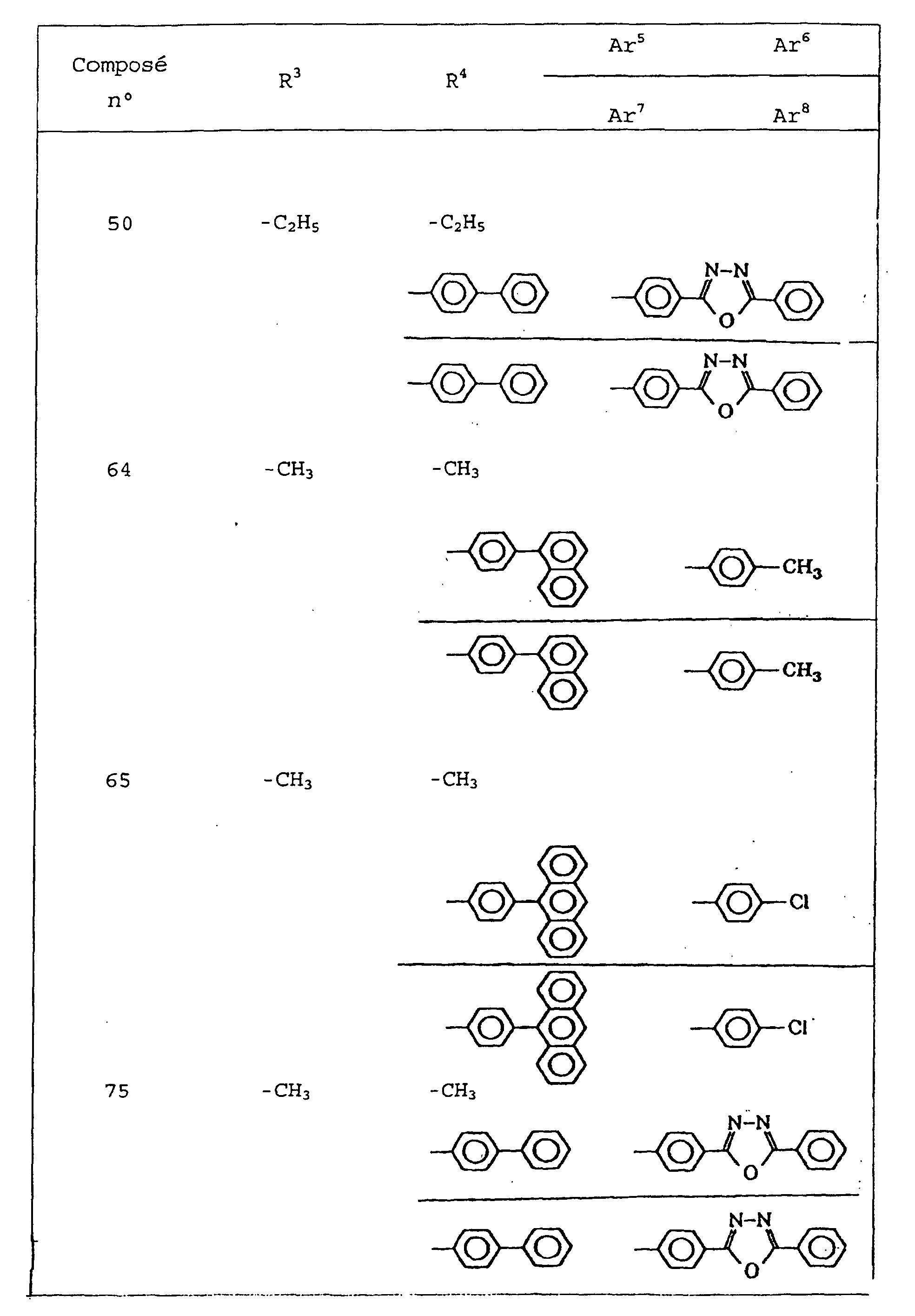

8. Dispositif luminescent comprenant une paire d'électrodes et une couche d'un composé organique disposée entre ces électrodes, la couche de composé organique comprenant un composé suivant l'une quelconque des revendications précédentes.

9. Dispositif luminescent suivant la revendication 8, dans lequel l'épaisseur de la couche de composé organique est inférieure à 2 µm.

10. Dispositif luminescent suivant la revendication 9, dans lequel l'épaisseur de la couche de composé organique est comprise dans l'intervalle de 0,05 à 0,5 µm.

11. Dispositif luminescent suivant la revendication 8, dans lequel la couche de composé organique est utilisée comme couche de transport de lacunes ou comme couche luminescente.

12. Dispositif luminescent comprenant une paire d'électrodes et une couche d'un composé organique disposée entre ces électrodes, la couche de composé organique comprenant un composé suivant l'une quelconque des revendications 1 à 4 et un composé suivant l'une quelconque des revendications 5 à 7.
